# EUROPÄISCHE PATENTSCHRIFT

(11) **EP 4 374 184 B1**
(45) Veröffentlichungstag und Bekanntmachung des Hinweises auf die Patenterteilung: **23.09.2026**
(21) Anmeldenummer: 22785923.8
(22) Anmeldetag: 22.08.2022
(51) Int. Cl.: G01R 33/34, G01R 33/3415

(54) **LOKALSPULE UND MAGNETRESONANZVORRICHTUNG MIT EINEM SICHERHEITSMECHANISMUS ZUR VERHINDERUNG EINER KOLLISION MIT EINEM PATIENTEN**
LOCAL COIL AND MAGNETIC RESONANCE APPARATUS HAVING A SAFETY MECHANISM FOR PREVENTING A COLLISION WITH A PATIENT
BOBINE LOCALE ET DISPOSITIF À RÉSONANCE MAGNÉTIQUE COMPRENANT UN MÉCANISME DE SÉCURITÉ POUR EMPÊCHER UNE COLLISION AVEC UN PATIENT

(30) Priorität: 17.09.2021 DE 102021210304; 28.02.2022 DE 102022202052
(43) Veröffentlichungstag der Anmeldung: 29.05.2024
(73) Patentinhaber: Siemens Healthineers AG, 91301 Forchheim (DE); Dentsply Sirona Inc., York, PA 17401 (US); Sirona Dental Systems GmbH, 64625 Bensheim (DE)
(72) Erfinder: GREISER, Andreas, 91054 Erlangen (DE); GEISSNER, Marco, 97230 Estenfeld (DE); ODOJ, Florian, 97209 Veitshöchheim (DE); ULRICI, Johannes, 64285 Darmstadt (DE)
(74) Vertreter: Müller Schupfner & Partner Patent- und Rechtsanwaltspartnerschaft mbB (Muc)
(86) Internationale Anmeldenummer: PCT/EP2022/073268
(87) Internationale Veröffentlichungsnummer: WO 2023/041288

(56) Entgegenhaltungen:
- DE-B4- 102011 075 454
- US-A- 5 307 806
- ANONYM: "Mandibula 15-Kanal Dental Spule - Noras MRI Products", 2017, Online, pages 1 - 74, XP055980776, Retrieved from the Internet <URL:https://docplayer.org/132670679-Mandibula-15-kanal-dental-spule-1-5t-si-t-si.html> [retrieved on 20221114]

## Beschreibung

Die Erfindung betrifft eine Lokalspule, umfassend zumindest eine Antenne, ein Basiselement, ein Halteelement, einen ersten Führungsmechanismus und einen zweiten Führungsmechanismus, wobei die zumindest eine Antenne dazu ausgebildet ist, hochfrequente Signale in einem Frequenz- und Leistungsbereich einer Magnetresonanzmessung zu empfangen und wobei die zumindest eine Antenne mechanisch mit dem Halteelement verbunden ist, wobei das Basiselement dazu ausgebildet ist, das Halteelement mit der zumindest einen Antenne in einer anwendungsgemäßen Position an einer diagnostisch relevanten Körperregion eines Patienten zu halten, wobei der erste Führungsmechanismus mechanisch mit dem Basiselement und dem Halteelement verbunden ist und dazu ausgebildet ist, das Halteelement veränderlich gegenüber dem Basiselement zu positionieren und wobei der zweite Führungsmechanismus mechanisch mit dem Halteelement und der zumindest einen Antenne verbunden ist und dazu ausgebildet ist, die zumindest eine Antenne veränderlich gegenüber dem Halteelement zu positionieren. Die Erfindung betrifft ferner eine Magnetresonanzvorrichtung mit einer erfindungsgemäßen Lokalspule.

Erkrankungen der Zähne und des Zahnhalteapparats, wie z. B. Karies oder Parodontitis, werden heutzutage üblicherweise mit röntgenbasierten Bildgebungsverfahren diagnostiziert. Dabei kommen vor allem konventionelle oder digitale Röntgen-Projektionsverfahren, sowie kürzlich auch dreidimensionale Röntgenverfahren, zum Einsatz. Ein Beispiel für ein dreidimensionales Röntgenverfahren stellt die digitale Volumentomographie dar, welche für eine Bildgebung von Zähnen und des Viscerocraniums eingesetzt werden kann.

Ein großer Nachteil von Röntgenverfahren ist die Notwendigkeit des Einsatzes von ionisierender Strahlung für die Bildgebung. Ein Bildgebungsverfahren, welches ionisierende Strahlen vermeidet, stellt die Magnetresonanztomografie dar. Diese ermöglicht typischerweise einen besseren Weichgewebekontrast als Röntgenverfahren und unterstützt standardmäßig eine dreidimensionale Bildgebung eines Untersuchungsobjekts. Weiterhin ermöglicht die Magnetresonanztomografie eine Bildgebung von Zysten sowie eine Erkennung einer Degradation von Dentin, bevor dies durch ein Röntgenverfahren erkennbar wird. Die Magnetresonanztomografie stellt somit eine potenzielle Alternative zu bekannten Röntgenverfahren bei der Bildgebung einer Gebissregion und/oder einer Kieferregion sowie der Diagnose von Zahnerkrankungen des Untersuchungsobjekts dar.

Die Magnetresonanztomografie ist ein bekanntes Bildgebungsverfahren, mit welchem Magnetresonanzbilder eines Inneren des Untersuchungsobjekts erzeugt werden können. Bei der Durchführung einer Magnetresonanzbildgebung wird das Untersuchungsobjekt üblicherweise in einem starken, statischen und homogenen Grundmagnetfeld (B0-Magnetfeld) einer Magnetresonanzvorrichtung positioniert. Das Grundmagnetfeld kann magnetische Feldstärken von 0,2 Tesla bis 7 Tesla aufweisen, sodass sich Kernspins des Untersuchungsobjekts entlang des Grundmagnetfeldes ausrichten. Um sogenannte Kernspinresonanzen auszulösen, werden hochfrequente Signale, sogenannte Anregungsimpulse (B1-Magnetfeld), in das Untersuchungsobjekt eingestrahlt. Jeder Anregungsimpuls bewirkt eine Abweichung einer Magnetisierung bestimmter Kernspins des Untersuchungsobjekts von dem Grundmagnetfeld um einen Betrag, welcher auch als Flipwinkel bekannt ist. Ein Anregungsimpuls kann dabei ein magnetisches Wechselfeld mit einer Frequenz aufweisen, welche der Larmorfrequenz bei der jeweiligen statischen Magnetfeldstärke entspricht. Die angeregten Kernspins können eine rotierende und abklingende Magnetisierung (Kernspinresonanz) aufweisen, welche sich mittels spezieller Antennen als Magnetresonanzsignal erfassen lässt. Zur räumlichen Kodierung der Kernspinresonanzen des Untersuchungsobjekts können dem Grundmagnetfeld magnetische Gradientenfelder überlagert werden.

Die empfangenen Magnetresonanzsignale werden typischerweise digitalisiert und als komplexe Werte in einer k-Raum-Matrix gespeichert. Diese k-Raum-Matrix kann als Grundlage für eine Rekonstruktion von Magnetresonanzbildern sowie eine Bestimmung von Spektroskopiedaten verwendet werden. Die Rekonstruktion eines Magnetresonanzbilds erfolgt typischerweise mittels einer mehrdimensionalen Fourier-Transformation der k-Raum-Matrix.

Die Magnetresonanztomografie eignet sich aufgrund der Vermeidung von ionisierender Strahlung insbesondere für eine kontinuierliche diagnostische Überwachung von Zahnerkrankungen und/oder einer Zahnentwicklung im Rahmen einer longitudinalen Bildgebungsstudie. Bei longitudinalen Bildgebungsstudien wird üblicherweise eine Mehrzahl von Bildgebungsuntersuchungen durchgeführt, um eine Progression einer Erkrankung oder einen Erfolg einer therapeutischen Behandlung über einen vorbestimmten Zeitraum zu bestimmen. Diagnostisch relevante Bereiche der Kieferregion eines Patienten, wie z. B. eine Mundhöhle, ein Gebiss, ein Zahnbogen oder ein Zahn, stellen jedoch ein geringes Volumen bereit, welches für eine Erzeugung von Magnetresonanzsignalen zur Verfügung steht. Ferner weisen konventionelle Volumen- und Oberflächenspulen, wie z. B. Kopfspulen und Auflegespulen, einen relativ großen Abstand zu der Kieferregion des Patienten auf. Ein großer Abstand kann jedoch ein Signal-zu-Rausch Verhältnis von erfassten Magnetresonanzsignalen erhöhen und somit eine Qualität von daraus rekonstruierten Magnetresonanzbildern des Gebisses des Patienten reduzieren. Für eine klinische Anwendung ist es außerdem von Vorteil, wenn sich die Spule wiederholbar und zeiteffizient in unmittelbarere Nähe an der Kieferregion des Patienten positionieren lässt. Aufgrund variierender Patientenanatomien sind aufgrund der unmittelbaren Nähe zu dem Patienten insbesondere auch Sicherheitsaspekte zu berücksichtigen.

Die Schrift "Mandibula 15-Kanal Dental Spule - Noras MRI Products", Internet, 2017, Seiten 1-74, XP055980776, URL: https://docplayer.org/132670679-Mandibula-15-kanal-dental-spule-1-5t-si-t-si.html, beschreibt eine 15-Kanal Dental Spule zur Bildgebung von Kiefer, Zähnen und Pulpa.

Die Druckschrift DE 10 2011 075454 B4 beschreibt eine Lokalspule für ein Magnetresonanztomographiesystem, welche ein Oberteil und ein Unterteil aufweist, wobei das Oberteil relativ zu dem Unterteil klappbar ist.

Es ist daher eine Aufgabe der Erfindung, eine Lokalspule bereitzustellen, welche ein Aufnehmen von Magnetresonanzbildern mit hoher Qualität ermöglicht, ohne die Sicherheit des Patienten zu kompromittieren.

Diese Aufgabe wird durch die Gegenstände der unabhängigen Patentansprüche erfindungsgemäß gelöst. Vorteilhafte Ausführungsformen und zweckmäßige Weiterbildungen sind Gegenstand der Unteransprüche.

Die erfindungsgemäße Lokalspule umfasst zumindest eine Antenne, ein Basiselement, ein Halteelement, einen ersten Führungsmechanismus, einen zweiten Führungsmechanismus und einen Sicherheitsmechanismus.

Die zumindest eine Antenne ist dazu ausgebildet, hochfrequente Signale in einem Frequenz- und Leistungsbereich einer Magnetresonanzmessung zu empfangen. Eine Antenne kann ein Koppelelement zwischen in Signalleitern geführten und ungeführten, d. h. in einem Freiraum befindlichen, elektromagnetischen Wellen darstellen. Die zumindest eine Antenne ist insbesondere dazu ausgebildet, elektromagnetische Wellen im Bereich einer Magnetresonanzfrequenz eines magnetresonanzaktiven Atomkerns zu empfangen. Für Magnetresonanzmessungen relevante elektromagnetische Wellen können hochfrequente Signale (Magnetresonanzsignale) darstellen, welche Frequenzen zwischen 1 und 500 MHz, vorzugsweise zwischen 10 und 300 MHz, umfassen. Die Magnetresonanzsignale üblicher zu untersuchender Atomkerne können eine geringe Leistung von einigen Mikrowatt bis mehrere Milliwatt aufweisen.

Ein Signalleiter ist vorzugsweise ein elektrisch leitender Draht. Der Draht des Signalleiters kann einen ovalen oder polygonalen Querschnitt aufweisen und dazu geeignet sein, die oben angegebenen Leistungen kontinuierlich zu übertragen. Es ist ebenso vorstellbar, dass der Signalleiter als eine Leiterbahn auf einer Leiterplatte ausgeführt ist und einen annähernd rechteckigen Querschnitt aufweist. Der Signalleiter kann aus Kupfer bestehen. Es sind aber auch andere elektrisch leitende Metalle, wie z. B. Gold, Aluminium und dergleichen, vorstellbar.

Selbstverständlich kann die erfindungsgemäße Lokalspule mehrere Antennen umfassen. Die Antennen können dabei voneinander beabstandet, aneinander angrenzend oder teilweise überlappend angeordnet sein. Die Antennen können ferner in Form eines Gitters oder einer Matrix angeordnet sein.

In einer bevorzugten Ausführungsform weist die Lokalspule eine Anzahl von Antennen auf, welche dazu ausgebildet sind, Magnetresonanzsignale aus der diagnostisch relevanten Körperregion, insbesondere einer Kopfregion oder einer Kieferregion des Patienten, zu empfangen. Die zumindest eine Antenne kann mechanisch mit einer Trägerstruktur verbunden sein und von dieser getragen oder gehalten werden. Es ist ebenso vorstellbar, dass die zumindest eine Antenne in die Trägerstruktur integriert oder eingebettet ist. Die Trägerstruktur kann ein Material aufweisen, welches dazu ausgebildet ist, einen Berührschutz für den Patienten bereitzustellen und/oder einer Kontur der diagnostisch relevanten Körperregion des Patienten nachgeformt zu werden.

Es ist vorstellbar, dass die erfindungsgemäße Lokalspule zumindest eine Antenne aufweist, welche dazu ausgelegt ist, ein hochfrequentes Signal in eine Richtung des Untersuchungsobjekts, wie z. B. eine Kieferregion des Patienten, auszusenden. Das von der zumindest einen Antenne ausgesandte, hochfrequente Signal kann in Abhängigkeit des Grundmagnetfelds einer Magnetresonanzvorrichtung beispielsweise in einem Leistungsbereich von wenigen Watt bis mehreren Kilowatt liegen. Das von der zumindest einen Antenne ausgesandte, hochfrequente Signal kann insbesondere ein B1-Magnetfeld darstellen. Ein Teil der Lokalspule mit der zumindest einen Antenne kann beispielsweis eine Sendeeinheit der Lokalspule darstellen.

Die zumindest eine Antenne ist mechanisch mit dem Halteelement verbunden. Das Halteelement kann ein beliebiges Trägerelement darstellen, welches dazu ausgebildet ist, die zumindest eine Antenne in einer vorbestimmten relativen Position zu der diagnostisch relevanten Körperregion des Patienten zu halten. Vorzugsweise ist das Halteelement dazu ausgebildet, die zumindest eine Antenne in grober Weise gegenüber dem Patienten zu positionieren und/oder auszurichten. Eine genaue Positionierung und/oder Ausrichtung der zumindest einen Antenne gegenüber der diagnostisch relevanten Körperregion des Patienten erfolgt bevorzugt mittels des zweiten Führungsmechanismus.

Das Basiselement ist dazu ausgebildet, das Halteelement mit der zumindest einen Antenne in einer anwendungsgemäßen Position an einer diagnostisch relevanten Körperregion eines Patienten zu halten. Das Basiselement ist hierfür vorzugsweise mit einer Komponente einer Magnetresonanzvorrichtung, wie z. B. einem Patiententisch und/oder einer Patientenlagerungsvorrichtung, mechanisch verbunden. Das Basiselement kann beispielsweise eine Positionierungseinheit aufweisen, welche dazu ausgebildet ist, das Basiselement und/oder das Halteelement mit der zumindest einen Antenne relativ zu Magnetresonanzvorrichtung und/oder dem Patienten zu positionieren. Das Basiselement kann die diagnostisch relevante Körperregion des Patienten in einer anwendungsgemäßen Position an dem Patienten an zwei gegenüberliegenden Seiten flankieren oder zumindest teilweise umschließen. Vorzugsweise ist das Basiselement dazu ausgebildet, eine Bewegung der diagnostisch relevanten Körperregion des Patienten während einer Bildgebungsuntersuchung zu reduzieren oder zu minimieren.

Der erste Führungsmechanismus ist mechanisch mit dem Basiselement und dem Halteelement verbunden und dazu ausgebildet, das Halteelement veränderlich gegenüber dem Basiselement zu positionieren. Der erste Führungsmechanismus kann ein beliebiges mechanisches Prinzip umfassen, welches ein relatives Bewegen des Halteelements gegenüber dem Basiselement ermöglicht. Vorzugsweise umfasst der erste Führungsmechanismus ein Gelenk, insbesondere einen Schwenk- oder Klappmechanismus, ein Schienensystem, eine Linearführung oder dergleichen. Der erste Führungsmechanismus ist vorzugsweise dazu ausgebildet, ein besonders einfaches und/oder zeiteffizientes Überführen des Halteelements von einer Öffnungsposition in eine Schließposition zu ermöglichen.

Der zweite Führungsmechanismus ist mechanisch mit dem Halteelement und der zumindest einen Antenne verbunden und dazu ausgebildet, die zumindest eine Antenne veränderlich gegenüber dem Halteelement zu positionieren. Vorzugsweise ist der zweite Führungsmechanismus dazu ausgebildet, ein genaues Positionieren und/oder Ausrichten der zumindest einen Antenne gegenüber der diagnostisch relevanten Körperregion zu ermöglichen. Der zweite Führungsmechanismus kann hierfür z. B. einen Schraubmechanismus, einen Klemmmechanismus, einen Rastermechanismus, ein Teleskopsystem, ein Schienensystem, aber auch ein Gelenk, ein Scharnier und/oder ein vergleichbares mechanisches Prinzip umfassen.

Insbesondere stellen der erste Führungsmechanismus und/oder der zweite Führungsmechanismus eine Führung für das Halteelement und/oder die zumindest eine Antenne bereit. Dies kann bedeuten, dass eine Bewegung des Halteelements mittels des ersten Führungsmechanismus und/oder eine Bewegung der zumindest einen Antenne mittels des zweiten Führungsmechanismus auf eine vorbestimmte Anzahl von Raumrichtungen und/oder Drehrichtungen beschränkt ist. Beispielsweise kann das Positionieren des Halteelements gegenüber dem Basiselement mittels des ersten Führungsmechanismus auf ein Bewegen des Halteelements entlang zwei entgegengesetzter Drehrichtungen beschränkt sein. Das Positionieren der zumindest einen Antenne gegenüber dem Haltelement kann dagegen auf ein Bewegen entlang einer Geraden, einer Ebene, einer Geraden und einer Ebene, aber auch zweier Ebenen, insbesondere zweier orthogonaler Ebenen, beschränkt sein.

Der Sicherheitsmechanismus ist dazu ausgebildet, eine Kollision der zumindest einen Antenne mit dem Patienten, insbesondere der diagnostisch relevanten Körperregion des Patienten, bei einem Überführen des Halteelements von einer Öffnungsposition in eine Schließposition mittels des ersten Führungsmechanismus zu verhindern. Vorzugsweise ist der Sicherheitsmechanismus dazu ausgebildet, eine relative Bewegung des Halteelements zu dem Basiselement zu begrenzen und/oder eine Anpassung einer relativen Position der zumindest einen Antenne zu dem Halteelement vorzunehmen oder zu initiieren. Es ist vorstellbar, dass der Sicherheitsmechanismus dazu ausgebildet ist, eine Kollision der zumindest einen Antenne mit der diagnostisch relevanten Körperregion des Patienten automatisch, insbesondere auch bei einer Fehlbedienung durch einen Nutzer, zu verhindern. Der Sicherheitsmechanismus kann weiterhin dazu ausgelegt sein, eine Mehrzahl von Szenarien einer potentiellen Fehlbenutzung der Lokalspule, welche eine Kollision der zumindest einen Antenne mit der diagnostisch relevanten Körperregion zur Folge haben, abzusichern oder zu verhindern.

Eine Kollision kann durch ein ungewünschtes Zusammenstoßen oder Zusammenführen der zumindest einen Antenne mit der diagnostisch relevanten Körperregion des Patienten charakterisiert sein. Eine Kollision kann eine Bewegung der zumindest einen Antenne in Richtung der diagnostisch relevanten Körperregion des Patienten voraussetzen. Es ist insbesondere vorstellbar, dass die Kollision einen Kontakt, eine Kraftwirkung und/oder eine Kraftübertragung zwischen der einen Antenne und der diagnostisch relevanten Körperregion des Patienten umfasst. Als Kollision sollen hierbei jedoch nur jene Bewegungen, Kontakte und/oder Kraftwirkungen gelten, welche durch eine Bewegung des Halteelements mittels des ersten Führungsmechanismus verursacht werden. Ein Kontakt zwischen der zumindest einen Antenne und der diagnostisch relevanten Körperregion, welcher allein durch eine Bewegung der zumindest einen Antenne mittels des zweiten Führungsmechanismus hergestellt wird, gilt somit als gewünscht und fällt nicht unter die oben gegebene Definition einer Kollision.
Eine Öffnungsposition des Halteelements kann durch eine maximale oder vorbestimmte Auslenkung des Halteelements gegenüber dem Basiselement mittels des ersten Führungsmechanismus charakterisiert sein. Die Öffnungsposition kann insbesondere eine "Ladeposition" darstellen, welche ein Positionieren der diagnostisch relevanten Körperregion des Patienten in einer anwendungsgemäßen, relativen Position zu dem Basiselement ermöglicht. Eine Schließposition des Halteelements kann hingegen durch eine minimale Auslenkung des Haltelements gegenüber dem Basiselement mittels des ersten Führungsmechanismus charakterisiert sein. Die Schließposition kann insbesondere eine "Untersuchungsposition" darstellen, welche durch eine anwendungsgemäße, relative Position des Haltelements zu der diagnostisch relevanten Körperregion des Patienten während einer Magnetresonanzmessung charakterisiert ist. Ein Überführen des Halteelements von der Öffnungsposition in die Schließposition kann insbesondere ein Zusammenführen des Halteelements und des Basiselements umfassen. Es ist vorstellbar, dass das Haltelement bei dem Zusammenführen mittels des ersten Führungsmechanismus in einer dem Basiselement zugewandten Richtung bewegt oder ausgelenkt wird.

Durch das Bereitstellen der erfindungsgemäßen Lokalspule lässt sich ein Risiko einer Kollision der zumindest einen Antenne mit der diagnostisch relevanten Körperregion des Patienten bei einem Positionieren des Haltelements und/oder der zumindest einen Antenne in der anwendungsgemäßen Position am Patienten vermeiden. Weiterhin lässt sich eine Sicherheit einer Handhabung der erfindungsgemäßen Lokalspule mittels eines erfindungsgemäßen Sicherheitsmechanismus erhöhen. Dadurch kann ein Positionieren der Lokalspule an der diagnostisch relevanten Körperregion auf vorteilhafte Weis auch durch weniger qualifiziertes Personal vorgenommen werden.

In einer Ausführungsform der erfindungsgemäßen Lokalspule ist der Sicherheitsmechanismus dazu ausgebildet, eine Bewegung des Halteelements mittels des ersten Führungsmechanismus bei dem Überführen des Halteelements von der Öffnungsposition in die Schließposition in Abhängigkeit einer Sicherheitsposition der zumindest einen Antenne zu dem Halteelement zu begrenzen.

Vorzugsweise ist der Sicherheitsmechanismus dazu ausgebildet, eine relative Bewegung des Halteelements zu dem Basiselement in Richtung des Basiselements zu verhindern oder zu blockieren, wenn die relative Position der zumindest eine Antenne zu dem Halteelement einen vorbestimmten Grenzwert überschreitet. Ein Begrenzen der Bewegung des Haltelements mittels des ersten Führungsmechanismus kann ein Abdämpfen, aber insbesondere ein Sperren oder ein Blockieren, der relativen Bewegung des Haltelements gegenüber dem Basiselement bedeuten. Der vorbestimmte Grenzwert kann beispielweise durch eine Sicherheitsposition der zumindest einen Antenne gegenüber dem Halteelement charakterisiert sein. Die Sicherheitsposition ist vorzugsweise durch einen vorbestimmten Abstand eines ersten Bezugspunkts der zumindest einen Antenne zu einem zweiten Bezugspunkt des Halteelements definiert. Beispielsweise kann der vorbestimmte Abstand des ersten Bezugspunkts der zumindest einen Antenne zu dem zweiten Bezugspunkt des Halteelements so gewählt sein, dass eine Kollision der zumindest einen Antenne mit einem Patienten beim Überführen des Halteelements von der Öffnungsposition in die Schließposition statistisch unwahrscheinlich ist oder ausgeschlossen werden kann.

In einer bevorzugten Ausführungsform ist der erste Führungsmechanismus derart mechanisch mit dem zweiten Führungsmechanismus gekoppelt, dass eine Bewegung des Halteelements in Richtung des Basiselements bei einem Überschreiten des vorbestimmten Grenzwerts verhindert wird.

Durch den erfindungsgemäßen Sicherheitsmechanismus kann ein Zusammenführen des Halteelements und des Basiselements auf vorteilhafte Weise vermieden werden, wenn die aktuelle relative Position der zumindest einen Antenne zu dem Halteelement von der Sicherheitsposition der zumindest einen Antenne gegenüber dem Halteelement abweicht. Dadurch lässt sich eine Gefahr einer Verletzung des Patienten bei einem Zusammenführen des Halteelements und des Basiselements auf vorteilhafte Weise verhindern.

In einer bevorzugten Ausführungsform der erfindungsgemäßen Lokalspule weist der Sicherheitsmechanismus eine erste Rastkomponente auf, welche dazu ausgebildet ist, in eine zweite Rastkomponente des ersten Führungsmechanismus einzugreifen und das Überführen des Halteelements von der Öffnungsposition in die Schließposition zu verhindern.

Im Rahmen der folgenden Erläuterungen wird vereinfachend angenommen, dass die zweite Rastkomponente als ein Rasterprofil ausgestaltet ist. Die erste Rastkomponente kann folglich als ein beliebiges Rastelement ausgestaltet sein, welches dazu ausgebildet ist, mechanisch in das Rasterprofil einzugreifen, um eine relative Bewegung des Rastelements zu dem Rasterprofil in zumindest eine Raumrichtung zu verhindern oder zu sperren. Es ist jedoch ebenso vorstellbar, dass die erste Rastkomponente als ein Rasterprofil ausgestaltet ist. In diesem Fall kann die zweite Rastkomponente ein beliebiges Rastelement darstellen, welches dazu ausgebildet ist, mechanisch in das Rasterprofil einzugreifen, um eine relative Bewegung des Rastelements zu dem Rasterprofil in zumindest eine Raumrichtung zu verhindern oder zu sperren.

Das Rastelement ist vorzugsweise dazu ausgebildet, formschlüssig und/oder kraftschlüssig in das Rasterprofil des ersten Führungsmechanismus einzugreifen, um die relative Bewegung des Halteelements zu dem Basiselement in zumindest einer Raumrichtung zu begrenzen oder zu verhindern. Das Rastelement und das Rasterprofil können derart ausgestaltet sein, dass das Rastelement das Rasterprofil in einer ersten Bewegungsrichtung, wie z. B. einer Öffnungsbewegung des Halteelements (also einer Bewegung des Haltelements in einer dem Basiselement entgegengesetzten Richtung), passieren kann, während das Rastelement in einer zweiten Bewegungsrichtung, wie z. B. einer Schließbewegung des Halteelements (also einer Bewegung des Haltelements in Richtung des Basiselements), in das Rasterprofil eingreift und eine weitere Bewegung verhindert. Das Rastelement kann an einem elastischen Element, wie z. B. einer mechanischen Feder oder einem Elastomer, gelagert sein. Es ist ebenso vorstellbar, dass das Rastelement selbst ein elastisches Material aufweist und/oder (elastisch) verformbar ist.

Die erste Rastkomponente ist mechanisch mit einem Sicherungselement verbunden, wobei die erste Rastkomponente mittels des Sicherungselements mechanisch von der zweiten Rastkomponente des ersten Führungsmechanismus trennbar ist, um das Überführen des Halteelements von der Öffnungsposition in die Schließposition freizugeben.

Das Sicherungselement kann einen Mechanismus umfassen, welcher dazu ausgebildet ist, eine Position, eine Form und/oder eine Ausrichtung des Rastelements und/oder des elastischen Elements zu verändern, um das Rastelement manuell oder automatisiert von dem Rasterprofil des ersten Führungsmechanismus zu trennen. Der Mechanismus kann insbesondere einen Klammermechanismus, einen Zugmechanismus, einen Federmechanismus, einen Biegemechanismus und/oder einen Stellmechanismus aufweisen, welche als ein Teil des Rastelements ausgebildet und/oder mechanisch mit dem Rastelement gekoppelt sind. Beispielsweise kann das Rastelement derart an dem Sicherungselement gelagert sein, dass das Rastelement in Abhängigkeit einer vorbestimmten Bewegung und/oder eine vorbestimmte Kraftwirkung des Sicherungselements auf das Rastelement von dem Rasterprofil trennbar ist. Das Sicherungselement kann ebenso ein elastisches Element gemäß einer oben beschriebenen Ausführungsform aufweisen oder als ein solches ausgestaltet sein.

Vorzugsweise ist das Sicherungselement mechanisch mit dem zweiten Führungsmechanismus gekoppelt, sodass die vorbestimmte Kraftwirkung und/oder die vorbestimmte Bewegung des Sicherungselements von der relativen Bewegung der zumindest einen Antenne zu dem Halteelement verursacht oder ausgelöst wird.
In einer weiteren Ausführungsform ist das Sicherungselement manuell durch einen Nutzer und/oder automatisch mittels einer elektrischen Schaltung betätigbar. Durch die Möglichkeit eines manuellen Betätigens des Sicherungselements kann eine Sperre der Bewegung des Halteelements in Richtung des Basiselements auf vorteilhafte Weise bewusst durch einen Nutzer aufgehoben werden. Durch ein Betätigen des Sicherungselements mittels einer elektrischen Schaltung lässt sich ein Aufheben der Sperre auf vorteilhafte Weise automatisieren. Beispielsweise kann hierbei ein Überwachen der relativen Position der zumindest einen Antenne zu dem Halteelement mittels eines oder mehrerer geeigneter Sensoren erfolgen.

In einer Ausführungsform der erfindungsgemäßen Lokalspule ist die erste Rastkomponente mittels des Sicherungselements mechanisch mit dem zweiten Führungsmechanismus gekoppelt. Die erste Rastkomponente ist mittels eines Bewegens der zumindest einen Antenne in eine vorbestimmte relative Position zu dem Halteelement mittels des zweiten Führungsmechanismus mechanisch von der zweiten Rastkomponente des ersten Führungsmechanismus trennbar, um das Überführen des Halteelements von der Öffnungsposition in die Schließposition freizugeben.

Das Sicherungselement kann dazu ausgebildet sein, eine Position, eine Form und/oder eine Ausrichtung des Rastelements zu verändern, um das Rastelement mechanisch von dem Rasterprofil des ersten Führungsmechanismus zu trennen. Das Sicherungselement kann hierfür insbesondere als ein elastisches Element gemäß einer oben beschriebenen Ausführungsform ausgestaltet sein. Vorzugsweise ist das Sicherungselement derart mechanisch mit dem zweiten Führungsmechanismus gekoppelt, dass das Rastelement bei Vorliegen einer vorbestimmten relativen Position der zumindest einen Antenne zu dem Halteelement automatisch von dem Rasterprofil des ersten Führungsmechanismus getrennt wird.

Ein Rastelement kann eine besonders einfach zu implementierende und/oder kostengünstige Lösung des erfindungsgemäßen Sicherheitsmechanismus darstellen. Weiterhin kann durch das Rastelement eine ausfallsichere und/oder robuste Lösung für das Verhindern einer Kollision der zumindest einen Antenne mit der diagnostisch relevanten Körperregion des Patienten bereitgestellt werden.

In einer Ausführungsform der erfindungsgemäßen Lokalspule ist der Sicherheitsmechanismus mechanisch mit dem ersten Führungsmechanismus und dem zweiten Führungsmechanismus gekoppelt und dazu ausgebildet, die zumindest eine Antenne unter Ausnutzung einer Kinematik einer relativen Bewegung des Halteelements zu dem Basiselement in eine Sicherheitsposition zu dem Halteelement zu überführen.

Der Sicherheitsmechanismus ist vorzugsweise als eine mechanische Kopplung zwischen dem ersten Führungsmechanismus und dem zweiten Führungsmechanismus ausgestaltet. Eine solche mechanische Kopplung kann insbesondere ein Getriebe mit einem oder mehreren Getriebekörpern und/oder Wellen umfassen. Vorzugsweise weist die mechanische Kopplung einen Leerlauf- oder Entkopplungsmechanismus auf, welcher dazu ausgebildet ist, die Überführung der zumindest eine Antenne in die Sicherheitsposition zu dem Halteelement auf eine Schließbewegung oder eine Öffnungsbewegung des Haltelements zu beschränken. Der Entkopplungsmechanismus kann weiterhin dazu ausgebildet sein, die zumindest eine Antenne mittels des Sicherheitsmechanismus bei der Öffnungsbewegung oder Schließbewegung in eine vorbestimmte relative Position gegenüber dem Halteelement zu überführen.

Die vorbestimmte relative Position der zumindest einen Antenne kann beispielsweise durch eine maximale Auslenkung bzw. einen maximalen Abstand der zumindest einen Antenne gegenüber dem Basiselement charakterisiert sein. Die zumindest eine Antenne kann sich hierbei in einer Anschlags- oder Endposition gegenüber dem Haltelement in einer von dem Basiselement abgewandten Richtung befinden. Es ist jedoch ebenso vorstellbar, dass die vorbestimmte relative Position durch eine zulässige Auslenkung der zumindest einen Antenne gegenüber dem Halteelement in Richtung des Basiselements charakterisiert ist. Vorzugsweise ist die zulässige Auslenkung dabei so gewählt, dass eine Kollision der zumindest einen Antenne mit der diagnostisch relevanten Körperregion bei einer Schließbewegung des Halteelements statistisch unwahrscheinlich ist oder ausgeschlossen werden kann. Die vorbestimmte relative Position der zumindest einen Antenne gegenüber dem Halteelement kann insbesondere mit der Sicherheitsposition der zumindest einen Antenne übereinstimmen.

Durch ein Bereitstellen einer mechanischen Kopplung zur Überführung der zumindest einen Antenne in die vorbestimmte relative Position zu dem Halteelement unter Ausnutzung einer Kinematik der relativen Bewegung des Halteelements und des Basiselements lassen sich ein zusätzlicher Antrieb und/oder ein zusätzlicher Arbeitsschritt der Positionierung der zumindest einen Antenne in der vorbestimmten relativen Position zu dem Halteelement auf vorteilhafte Weise einsparen.

In einer weiteren Ausführungsform der erfindungsgemäßen Lokalspule weist der zweite Führungsmechanismus ein Spannelement auf, welches dazu ausgebildet ist, infolge einer relativen Bewegung der zumindest einen Antenne zu dem Halteelement in Richtung des Basiselements elastisch verformt zu werden und eine der Bewegung entgegengerichtete Kraft auf die zumindest eine Antenne zu übertragen. Ein Spannelement kann ein beliebiges elastisches Element, wie z. B. eine mechanische Feder, ein Gummiband und/oder einen Körper aus einem elastischen Material, darstellen. Ein elastisches Material kann ein synthetisches oder natürliches Elastomer, wie z. B. Kautschuk oder ein synthetisches Polymer, aufweisen. Das Spannelement kann insbesondere aus einem Kunststoff oder einem Metall gefertigt sein.

Das Spannelement weist vorzugsweise eine mechanische Verbindung mit der zumindest einen Antenne und dem Haltelement auf. Das Spannelement kann derart mit der zumindest einen Antenne und dem Halteelement verbunden sein, dass das Spannelement bei einer relativen Bewegung der zumindest einen Antenne zu dem Haltelement in Richtung des Basiselements elastisch verformt wird. Durch die elastische Verformung können elastische Rückstellkräfte in dem Spannelement aufgebaut werden, welche der Bewegungsrichtung der zumindest einen Antenne entgegengerichtet sind.

Der Sicherheitsmechanismus weist eine erste Rastkomponente auf, welche mechanisch mit der zumindest einen Antenne verbunden ist und dazu ausgebildet ist, in eine zweite Rastkomponente des zweiten Führungsmechanismus einzugreifen und der Kraft des Spannelements entgegenzuwirken. Die erste Rastkomponente und die zweite Rastkomponente können hierbei gemäß einer oben beschriebenen Ausführungsform ausgeführt sein.

Die erste Rastkomponente ist mechanisch mit dem ersten Führungsmechanismus gekoppelt, wobei die erste Rastkomponente mittels einer vorbestimmten relativen Bewegung des Halteelements zu dem Basiselement mechanisch von der zweiten Rastkomponente des zweiten Führungsmechanismus trennbar ist, um ein Auslenken der zumindest einen Antenne in eine dem Basiselement abgewandte Richtung mittels des Spannelements zu ermöglichen. Die mechanische Kopplung des ersten Führungsmechanismus mit dem Rastelement kann beispielsweise als ein Sicherungselement gemäß einer oben beschriebenen Ausführungsform ausgestaltet sein. Vorzugsweise ist ein solches Sicherungselement dazu ausgebildet, das Rastelement bei einer Öffnungsbewegung des Halteelements von dem Rasterprofil des zweiten Führungsmechanismus zu trennen, wobei die zumindest eine Antenne durch die elastischen Rückstellkräfte des Spannelements in eine Ausgangsposition überführt wird.

Unter Anwendung des gleichen mechanischen Prinzips können das Spannelement und die mechanische Kopplung alternativ auch dazu ausgebildet sein, die zumindest eine Antenne in eine Ausgangsposition zurückzuführen, wenn eine Schließbewegung des Haltelements durchgeführt wird, also das Haltelement relativ zu dem Basiselement in Richtung des Basiselements bewegt wird.

Durch das Bereitstellen eines Spannelementes und eines entsprechenden Sicherheitsmechanismus kann auf vorteilhafte Weise gewährleistet werden, dass sich die zumindest eine Antenne nach einer Öffnungsbewegung des Halteelements in einer Ausgangsposition befindet. Weiterhin lässt sich durch eine rein mechanische Lösung für die automatisierte Rückstellung der zumindest einen Antenne ein zusätzlicher Energiebedarfs für elektrisch angetriebene Komponenten auf vorteilhafte Weise vermeiden.

In einer weiteren Ausführungsform der erfindungsgemäßen Lokalspule umfasst der Sicherheitsmechanismus ein Stellelement, welches dazu ausgebildet ist, eine Kraft auf die zumindest eine Antenne zu übertragen, um die zumindest eine Antenne relativ zu dem Halteelement in eine dem Basiselement abgewandte Richtung auszulenken. Das Stellelement kann beispielsweise mit einem pneumatischen, einem hydraulischen und/oder einem elektrischen Antrieb verbunden sein, welcher dazu ausgebildet ist, das Stellelement sowie die zumindest eine Antenne in eine Ausgangsposition und/oder die vorbestimmte relative Position zu dem Halteelement zu überführen. Vorzugsweise ist das Stellelement dazu ausgebildet, die zumindest eine Antenne entlang einer durch den zweiten Führungsmechanismus vorgegebenen Bewegungstrajektorie in eine dem Basiselement abgewandte Richtung zu transportieren oder zu bewegen.

Die Lokalspule weist ferner ein Arretierungselement auf, welches mechanisch mit dem ersten Führungsmechanismus gekoppelt ist und dazu ausgebildet ist, die Übertragung der Kraft auf die zumindest eine Antenne durch das Stellelement zu unterbrechen, wenn sich das Basiselement und das Haltelement in einer vorbestimmten relativen Position befinden. Das Arretierungselement kann beispielsweise als ein Anschlagselement, wie z. B. ein Stift, ein Bolzen, ein Rastelement, eine Platte, aber auch eine Armatur, ein Ventil oder dergleichen, ausgestaltet sein. Das Arretierungselement kann insbesondere dazu ausgebildet sein, eine Kraftwirkung des Antriebs auf das Stellelement zu unterbrechen.

Es ist ebenso vorstellbar, dass das Arretierungselement dazu ausgebildet ist, eine Bewegung der zumindest einen Antenne entlang der durch den zweiten Führungsmechanismus vorgegebenen Bewegungstrajektorie freizugeben. Die mechanische Kopplung zwischen dem Arretierungselement und dem ersten Führungsmechanismus kann gemäß einer oben beschriebenen Ausführungsform ausgestaltet sein. Vorzugsweise ist die mechanische Kopplung dazu ausgebildet, dass Arretierungselement in eine Öffnungs- oder Schließposition zu überführen, wenn das Haltelement in eine Schließposition gegenüber dem Basiselement geführt wird. Dabei kann die Auslenkung der zumindest einen Antenne oder die Kraftwirkung des Stellelements auf die zumindest eine Antenne durch die Überführung des Arretierungselements in die Öffnungs- oder Schließposition unterbrochen werden.

In einer alternativen Ausführungsform der erfindungsgemäßen Lokalspule umfasst der Sicherheitsmechanismus ein Stellelement, wobei das Stellelement dazu ausgebildet ist, eine Kraft auf das Halteelement zu übertragen, um eine relative Bewegung des Halteelements zu dem Basiselement zu begrenzen und/oder das Halteelement in eine dem Basiselement abgewandte Richtung auszulenken. Das Stellelement und der Antrieb können gemäß einer oben beschriebenen Ausführungsform ausgestaltet sein. Insbesondere kann der Antrieb als ein pneumatischer, ein hydraulischer oder ein elektrischer Antrieb ausgestaltet sein.

Die Lokalspule weist ferner ein Arretierungselement auf, welches mechanisch mit dem zweiten Führungsmechanismus gekoppelt ist und dazu ausgebildet ist, die Übertragung der Kraft auf das Halteelement durch das Stellelement zu unterbrechen, wenn sich die zumindest eine Antenne in einer vorbestimmten relativen Position zu dem Halteelement befindet. Die vorbestimmte relative Position der zumindest einen Antenne zu dem Halteelement ist vorzugsweise durch einen maximalen Abstand der zumindest einen Antenne gegenüber dem Basiselement und/oder einer maximalen Auslenkung der zumindest einen Antenne in einer dem Basiselement abgewandten Richtung gegenüber dem Halteelement charakterisiert.

In einer Ausführungsform der erfindungsgemäßen Lokalspule weist das Stellelement eine Fluidverbindung mit einem pneumatischen und/oder hydraulischen Antrieb auf, wobei das Arretierungselement dazu ausgebildet ist, die Fluidverbindung zwischen dem Stellelement und dem pneumatischen und/oder hydraulischen Antrieb zu unterbrechen. Das Arretierungselement kann beispielsweise als ein Ventil, insbesondere ein 3-WegeVentil, ausgestaltet sein.

In einer Ausführungsform ist das Ventil dazu ausgebildet, die Kraftwirkung des Stellelements auf die zumindest eine Antenne zu unterbrechen, wenn sich das Halteelement in einer Schließposition gegenüber dem Basiselement befindet. Die mechanische Kopplung zwischen dem ersten Führungsmechanismus und dem Ventil kann hierbei insbesondere als ein Stellantrieb für das Ventil ausgestaltet sein. Das Ventil kann mittels des Stellantriebs in eine Konfiguration überführt werden, welche die Fluidverbindung zu dem pneumatischen oder hydraulischen Antrieb unterbricht oder öffnet.

In einer alternativen Ausführungsform ist das Ventil dazu ausgebildet, die Kraftwirkung des Stellelements auf das Halteelement zu unterbrechen, wenn sich die zumindest eine Antenne in der vorbestimmten relativen Position zu dem Halteelement befindet.

Durch ein Bereitstellen eines Stellelements gemäß einer oben beschriebenen Ausführungsform lassen sich pneumatische und/oder hydraulische Systeme, welche üblicherweise bereits in einem Untersuchungsraum einer Magnetresonanzvorrichtung vorhanden sind, vorteilhaft für eine Realisierung des Sicherheitsmechanismus einsetzen. Ferner können Fluidleitungen, insbesondere Fluidleitungen pneumatischer Systeme, aus Materialien gefertigt sein, welche eine geringe oder eine vernachlässigbare Interaktion mit magnetischen und/oder hochfrequenten Feldern aufweisen. Daher lassen sich solche Fluidleitungen auf vorteilhafte Weise auch in einem Patientenaufnahmebereich oder einer Bildaufnahmeregion der Magnetresonanzvorrichtung verwenden, ohne eine Qualität von erfassten Magnetresonanzbildern zu beeinflussen.

In einer weiteren Ausführungsform der erfindungsgemäßen Lokalspule weist der Sicherheitsmechanismus ein Stellelement mit einem Antrieb auf, wobei das Stellelement mechanisch mit der zumindest einen Antenne oder dem Halteelement verbunden ist und wobei der Antrieb dazu ausgebildet ist, die zumindest eine Antenne oder das Halteelement in Abhängigkeit eines Steuersignals mittels des Stellelements in eine dem Basiselement abgewandte Richtung auszulenken. Der Antrieb ist vorzugsweise außerhalb des Patientenaufnahmebereichs oder der Bildaufnahmeregion der Magnetresonanzvorrichtung positioniert. Der Antrieb kann mittels einer Fluidleitung, aber auch einer elektrischen und/oder einer mechanischen Kopplung, mit dem Stellelement verbunden sein. Es ist vorstellbar, dass der Antrieb in die Lokalspule und/oder die Patientenlagerungsvorrichtung integriert ist.

Der Sicherheitsmechanismus kann ferner eine Steuereinheit umfassen, welche dazu ausgebildet ist, das Steuersignal an den Antrieb auszugeben oder zu übermitteln. Es ist vorstellbar, dass die Steuereinheit dazu ausgebildet ist, das Steuersignal in Abhängigkeit einer Öffnungsposition und/oder einer Schließposition des Halteelements und des Basiselements bereitzustellen. Die Steuereinheit kann hierfür eine Signalverbindung mit einem Sensor aufweisen, welcher dazu ausgebildet ist, eine relative Position des Halteelements zu dem Basiselement, insbesondere eine Öffnungsposition und/oder eine Schließposition des Halteelements, zu ermitteln. In einem einfachen Beispiel kann der Sensor als ein Schalter, ein Kontakt, ein Relais oder dergleichen ausgestaltet sein. Solche Sensoren sind insbesondere dazu geeignet, ein Vorliegen des Halteelements in einer Endposition oder Anschlagsposition relativ zu dem Basiselement zu ermitteln. Der Sensor kann aber auch komplexere Messverfahren, wie z. B. eine LASER Abstandsmessung zwischen dem Halteelement und dem Basiselement oder ein Ermitteln einer Deformation eines faser-optischen Sensors bei Erreichen der Schließposition und/oder Öffnungsposition des Halteelements, umfassen. Der Sensor kann dazu geeignet sein, eine endliche Anzahl von vorbestimmten relativen Positionen des Halteelements gegenüber dem Basiselement zu ermitteln. Es ist aber ebenso vorstellbar, dass der Sensor dazu ausgebildet ist, die relative Position des Halteelements gegenüber dem Basiselement kontinuierlich oder in diskreten Zeitschritten zu ermitteln.

In einer Ausführungsform weist die Steuereinheit eine Signalverbindung mit einer Steuereinheit und/oder einer Recheneinheit der Magnetresonanzvorrichtung auf. Es ist ebenso vorstellbar, dass die Steuereinheit mit der Steuereinheit der Magnetresonanzvorrichtung übereinstimmt oder in diese integriert ist. Das Steuersignal kann entsprechend in Abhängigkeit eines Sensors der Magnetresonanzvorrichtung und/oder eines Parameters einer Magnetresonanzmessung ausgegeben werden. Ein Parameter einer Magnetresonanzmessung kann beispielsweise ein Bildgebungsparameter, aber auch ein beliebiger Parameter, welcher einen Ablauf oder einen Fortschritt der Magnetresonanzmessung charakterisiert, umfassen.

Durch das Bereitstellen eines erfindungsgemäßen elektrischen Antriebs und einer Steuereinheit lassen sich Maßnahmen zur Verhinderung der Kollision der zumindest einen Antenne mittels des Sicherheitsmechanismus auf vorteilhafte Weise automatisieren. Dadurch lässt sich ein Risiko einer Fehlbedienung der erfindungsgemäßen Lokalspule durch einen Nutzer vorteilhaft reduzieren oder vollständig vermeiden.

In einer Ausführungsform sind der erste Führungsmechanismus und/oder der zweite Führungsmechanismus als eine Linearführung, eine Teleskopführung, ein Gelenk, ein Scharnier oder ein Stecksystem ausgestaltet. Ein Stecksystem kann mehrere Komponenten aufweisen, welche in einem zerlegten bzw. demontierten Zustand separat voneinander vorliegen. In einer bevorzugten Ausführungsform ist das Basiselement mittels einer Positionierungseinheit mechanisch mit der Patientenlagerungsvorrichtung verbunden, wobei das Halteelement mit der zumindest einen Antenne in einer im Wesentlichen vertikalen Richtungen führbar oder steckbar mit dem Basiselement verbindbar ist. Das Halteelement kann aber auch in einer im Wesentlichen horizontalen Richtung oder in einer zu der vertikalen Richtung geneigten Richtung relativ zu dem Basiselement steckbar oder führbar sein.

Grundsätzlich können der erste Führungsmechanismus und/oder der zweite Führungsmechanismus einen beliebigen Mechanismus aufweisen, welcher eine relative Bewegung des Halteelements zu dem Basiselement und/oder der zumindest einen Antenne zu dem Halteelement auf eine oder mehrere vorbestimmte Bewegungstrajektorien zu begrenzen.

In einer bevorzugten Ausführungsform der erfindungsgemäßen Lokalspule weist der erste Führungsmechanismus einen Schwenkmechanismus auf, wobei das Halteelement mittels des Schwenkmechanismus um einen maximalen Winkel schwenkbar gegenüber dem Basiselement gelagert ist.

Der Schwenkmechanismus kann beispielsweise ein Scharnier, ein Gelenk, ein Drehlager, ein Gleitlager, ein Wälzlager, ein Rollenlager und/oder einen beliebigen anderen Mechanismus aufweisen, welcher dazu ausgebildet ist, das erste Element und/oder das zweite Element in einem Winkel gegenüber der Halterung und/oder der Patientenlagerungsvorrichtung zu schwenken. Vorzugsweise sind das erste Element und/oder das zweite Element bei anwendungsgemäßer Positionierung der Lokalspule an dem Patienten entlang einer Sagittalebene des Patienten schwenkbar. Es ist aber ebenso vorstellbar, dass das Halteelement annähernd parallel zu einer Frontalebene des Patienten schwenkbar ist. Das Halteelement und/oder die zumindest eine Antenne können beim Schwenken einem Abschnitt eines Kreisbogens folgen, welcher durch den Schwenkmechanismus bestimmt ist. Es ist vorstellbar, dass der Schwenkmechanismus gemäß einer oben beschriebenen Ausführungsform von dem Basiselement umfasst und/oder in dieses integriert ist.

In einer bevorzugten Ausführungsform weist der Schwenkmechanismus ein Rastelement und ein Rasterprofil auf. Das Rastelement kann gemäß einer oben beschriebenen Ausführungsform dazu ausgebildet sein, in das Rasterprofil einzugreifen und ein Schwenken des Halteelements entlang in Richtung des Basiselements zu verhindern. Das Rastelement ist vorzugsweise mechanisch mit dem zweiten Führungsmechanismus gekoppelt. Die mechanische Kopplung kann derart ausgestaltet sein, dass das Rastelement von dem Rasterprofil getrennt wird, wenn die zumindest eine Antenne mittels des zweiten Führungsmechanismus in eine vorbestimmte relative Position zu dem Haltelement bewegt wird.

Die Öffnungsposition ist durch den maximalen Winkel zwischen dem Basiselement und dem Halteelement charakterisiert. Der maximale Winkel ist so gewählt, dass ein Kopf des Patienten ungehindert in einer anwendungsgemäßen Relativposition zu der Lokalspule positioniert werden kann.

Der maximale Winkel kann in Abhängigkeit eines Aufbaus der Magnetresonanzvorrichtung und/oder der Patientenlagerungsvorrichtung zwischen 60° und 90°, 90° und 180° oder 180° und 270° betragen. Bei Magnetresonanzvorrichtungen, bei welchen der Patient bei der Magnetresonanzmessung eine stehende oder sitzende Körperhaltung einnimmt, beträgt der maximale Winkel vorzugsweise zwischen 60° und 90°. Es sind aber auch größere Werte des maximalen Winkels vorstellbar. Bei konventionellen Magnetresonanzvorrichtungen mit einem Patiententisch ist der maximale Winkel vorzugsweise kleiner als 180°.

Durch das Bereitstellen eines Schwenkmechanismus lässt sich das Halteelement auf besonders zeiteffiziente Weise an der diagnostisch relevanten Körperregion des Patienten positionieren und wieder vollständig aus einem Zugangsbereich des Patienten zu der Patientenlagerungsvorrichtung entfernen. Dadurch lässt sich ein Zeitaufwand, welcher mit einer anwendungsgemäßen Positionierung der Lokalspule an der diagnostisch relevanten Körperregion des Patienten verbunden ist, auf vorteilhafte Weise reduzieren.

In einer weiteren Ausführungsform weist die erfindungsgemäße Lokalspule eine zweite Antenne auf, wobei die zweite Antenne mechanisch mit dem Basiselement verbunden ist. Die zweite Antenne kann analog zu der zumindest einen Antenne ausgestaltet sein. Es ist vorstellbar, dass die zweite Antenne elektrisch von der zumindest einen Antenne getrennt ist oder elektrisch mit der zumindest einen Antenne verbunden ist.

Die zweite Antenne ist bei einer anwendungsgemäßen Positionierung des Patienten relativ zu dem Basiselement an einer von der zumindest einen Antenne abgewandten Seite des Patienten positioniert. Vorzugsweise ist die zweite Antenne in das Basiselement integriert bzw. eingebettet. Es ist ebenso vorstellbar, dass die zweite Antenne formschlüssig, kraftschlüssig und/oder stoffschlüssig mit dem Basiselement verbunden ist.

In einer bevorzugten Ausführungsform ist die erfindungsgemäße Lokalspule als eine Kopfspule oder eine Dentalspule ausgestaltet. Vorzugsweise ist das Basiselement der Dentalspule dazu ausgebildet, den Kopf des Patienten aufzunehmen. Bei einer anwendungsgemäßen Positionierung des Patienten relativ zu dem Basiselement kann die zweite Antenne an einer dorsalen Seite und/oder einem Scheitelbein des Patienten positioniert sein. Die erfindungsgemäße Lokalspule ermöglicht es somit auf vorteilhafte Weise, Magnetresonanzsignale aus einem größeren Volumen, insbesondere einer Kieferregion und/oder einer dorsalen Region des Kopfs des Patienten zu empfangen.

Die erfindungsgemäße Magnetresonanzvorrichtung umfasst eine Lokalspule gemäß einer oben beschriebenen Ausführungsform. In einer bevorzugten Ausführungsform ist die Lokalspule mechanisch mit einem Patiententisch und/oder einer Patientenlagerungsvorrichtung der Magnetresonanzvorrichtung verbunden. Die Lokalspule kann insbesondere eine Positionierungseinheit aufweisen, welche dazu ausgebildet ist, die Lokalspule relativ zu der Magnetresonanzvorrichtung und/oder einem Patienten zu positionieren. Die Lokalspule umfasst zumindest eine Antenne, ein Basiselement, ein Halteelement, einen ersten Führungsmechanismus, einen zweiten Führungsmechanismen und einen Sicherheitsmechanismus gemäß einer oben beschriebenen Ausführungsform. Die Magnetresonanzvorrichtung ist dazu ausgebildet, mittels der Lokalspule Magnetresonanzdaten einer diagnostisch relevanten Körperregion eines Patienten zu erfassen.

In einer Ausführungsform weist die Magnetresonanzvorrichtung eine Steuereinheit auf, welche mittels einer Signalverbindung mit einem Antrieb für ein Stellelement des Sicherheitsmechanismus verbunden ist. Vorzugsweise ist die Steuereinheit dazu ausgebildet, eine Information über den Fortschritt einer Magnetresonanzmessung mittels der Signalverbindung an eine Steuereinheit des Antriebs zu übermitteln. Es ist jedoch ebenso vorstellbar, dass die Steuereinheit dazu ausgebildet ist, ein Steuersignal an den Antrieb zu übertragen. Der Antrieb ist vorzugsweise dazu ausgebildet, eine Kraft auf ein Stellelement zu übertragen, welches mechanisch mit dem Halteelement oder der zumindest einen Antenne der Lokalspule verbunden ist. Der Sicherheitsmechanismus kann somit gewährleisten, dass ein relatives Positionieren des Haltelementes in Richtung des Basiselements vermieden wird, wenn eine relative Position der zumindest einen Antenne von einer vorbestimmten relativen Position der zumindest einen Antenne zu dem Halteelement abweicht. Es ist ebenso vorstellbar, dass der Sicherheitsmechanismus dazu ausgebildet ist, die zumindest eine Antenne in eine vorbestimmte relative Position zu dem Halteelement zu überführen, wenn das Halteelement in einer vorbestimmten relativen Position zu dem Basiselement positioniert wird. In einem weiteren Beispiel kann der Sicherheitsmechanismus ferner dazu ausgebildet sein, ein relatives Positionieren der zumindest einen Antenne gegenüber dem Halteelement zu ermöglichen, wenn das Halteelement in einer vorbestimmten relativen Position zu dem Basiselement positioniert wird.

Die erfindungsgemäße Magnetresonanzvorrichtung teilt dir Vorteile der erfindungsgemäßen Lokalspule.

Weitere Vorteile und Einzelheiten ergeben sich aus der nachfolgenden Beschreibung von Ausführungsbeispielen in Verbindung mit den Zeichnungen. Es zeigen in Prinzipdarstellung:
- Fig. 1: eine schematische Darstellung einer Ausführungsform einer erfindungsgemäßen Magnetresonanzvorrichtung,
- Fig. 2: eine Darstellung einer Ausführungsform einer erfindungsgemäßen Lokalspule,
- Fig. 3: eine mögliche Ausführungsform eines Sicherheitsmechanismus einer erfindungsgemäßen Lokalspule,
- Fig. 4: eine mögliche Ausführungsform eines Sicherheitsmechanismus einer erfindungsgemäßen Lokalspule,
- Fig. 5: eine mögliche Ausführungsform eines Sicherheitsmechanismus einer erfindungsgemäßen Lokalspule,
- Fig. 6: eine mögliche Ausführungsform eines Sicherheitsmechanismus einer erfindungsgemäßen Lokalspule,
- Fig. 7: eine mögliche Ausführungsform eines Sicherheitsmechanismus einer erfindungsgemäßen Lokalspule.

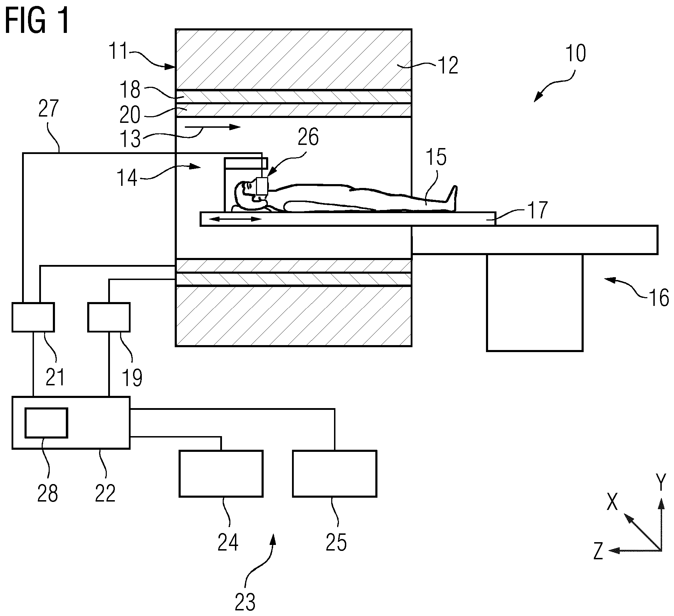

In Fig. 1 ist eine mögliche Ausführungsform einer erfindungsgemäßen Magnetresonanzvorrichtung 10 mit einer erfindungsgemäßen Lokalspule 26 schematisch dargestellt. Die Magnetresonanzvorrichtung 10 umfasst eine Magneteinheit 11, welche z. B. einen Permanentmagneten, einen Elektromagneten oder einen supraleitenden Hauptmagneten 12 zur Erzeugung eines starken und insbesondere homogenen Grundmagnetfelds 13 (B0-Magnetfeld) aufweist. Zudem umfasst die Magnetresonanzvorrichtung 10 einen Patientenaufnahmebereich 14 zu einer Aufnahme eines Patienten 15. Der Patientenaufnahmebereich 14 ist in dem vorliegenden Ausführungsbeispiel zylinderförmig ausgebildet und in einer Umfangsrichtung von der Magneteinheit 11 umgeben. Grundsätzlich sind jedoch auch von diesem Beispiel abweichende Ausbildungen des Patientenaufnahmebereichs 14 vorstellbar.

Der Patient 15 kann mittels einer Patientenlagerungsvorrichtung 16 der Magnetresonanzvorrichtung 10 in dem Patientenaufnahmebereich 14 positioniert werden. Die Patientenlagerungsvorrichtung 16 weist hierfür einen innerhalb des Patientenaufnahmebereichs 14 bewegbar ausgestalteten Patiententisch 17 auf. Die Magneteinheit 11 weist weiterhin eine Gradientenspule 18 zum Erzeugen von magnetischen Gradientenfeldern auf, welche für eine Ortskodierung während einer Magnetresonanzmessung verwendet wird. Die Gradientenspule 18 wird mittels einer Gradientensteuereinheit 19 der Magnetresonanzvorrichtung 10 angesteuert. Die Magneteinheit 11 kann weiterhin eine Hochfrequenzantenne umfassen, welche im vorliegenden Ausführungsbeispiel als fest in die Magnetresonanzvorrichtung 10 integrierte Körperspule 20 ausgebildet ist. Die Körperspule 20 ist zu einer Anregung von Atomkernen ausgelegt, die sich in dem von dem Hauptmagneten 12 erzeugten Grundmagnetfeld 13 befinden. Die Körperspule 20 wird von einer Hochfrequenzeinheit 21 der Magnetresonanzvorrichtung 10 angesteuert und strahlt hochfrequente Signale in einen Untersuchungsraum ein, der im Wesentlichen von einem Patientenaufnahmebereich 14 der Magnetresonanzvorrichtung 10 gebildet ist. Die Körperspule 20 kann weiterhin auch zu einem Empfangen von Magnetresonanzsignalen ausgebildet sein.

Für eine Steuerung des Hauptmagneten 12, der Gradientensteuereinheit 19 und der Hochfrequenzeinheit 21 weist die Magnetresonanzvorrichtung 10 eine Steuereinheit 22 auf. Die Steuereinheit 22 ist dazu ausgebildet eine Durchführung einer Sequenz, wie z. B. einer bildgebenden Gradientenechosequenz, einer TSE-Sequenz oder einer UTE-Sequenz, zu steuern. Zudem umfasst die Steuereinheit 22 eine Auswerteeinheit 28 zu einer Auswertung von digitalisierten Magnetresonanzsignalen, die während einer Magnetresonanzmessung erfasst werden.

Des Weiteren umfasst die Magnetresonanzvorrichtung 10 eine Benutzerschnittstelle 23, welche eine Signalverbindung mit der Steuereinheit 22 aufweist. Steuerinformationen, wie beispielsweise Bildgebungsparameter und rekonstruierte Magnetresonanzbilder, können auf einer Anzeigeeinheit 24, beispielsweise auf zumindest einem Monitor der Benutzerschnittstelle 23, für einen Nutzer angezeigt werden. Weiterhin weist die Benutzerschnittstelle 23 eine Eingabeeinheit 25 auf, mittels der Parameter einer Magnetresonanzbildgebung von dem Nutzer eingegeben werden können.

Ferner weist die Magnetresonanzvorrichtung 10 eine Lokalspule 26 auf, welche vorliegend an einem Kopf des Patienten 15 positioniert ist und Magnetresonanzsignale aus einem Volumen einer Kieferregion an die Magnetresonanzvorrichtung 10 überträgt. Die Lokalspule 26 weist vorzugsweise eine elektrische Anschlussleitung 27 auf, welche eine Signalverbindung mit der Hochfrequenzeinheit 21 und der Steuereinheit 22 bereitstellt. Die Lokalspule 26 kann aber auch mittels einer drahtlosen Signalverbindung mit der Magnetresonanzvorrichtung 10 verbunden sein. Ebenso wie die Körperspule 20 kann auch die Lokalspule 26 zu einer Anregung von Atomkernen und zu einem Empfangen von Magnetresonanzsignalen ausgebildet sein. Zum Aussenden von hochfrequenten Signalen wird eine Sendeeinheit der Lokalspule 26 von der Hochfrequenzeinheit 21 angesteuert. Die Lokalspule 26 kann den Kopf des Patienten 15 außenumfänglich entlang einer Längsachse des Patienten 15 umschließen. Die Sendeeinheit und/oder eine Empfangseinheit der Lokalspule 26 können insbesondere von einem Halteelement 33 getragen werden, welches relativ zu einem Basiselement der Lokalspule 26 positionierbar ist.

Die dargestellte Magnetresonanzvorrichtung 10 kann selbstverständlich weitere Komponenten umfassen, welche Magnetresonanzvorrichtungen üblicherweise aufweisen. Es ist ebenso vorstellbar, dass die Magnetresonanzvorrichtung 10 statt des zylinderförmigen Aufbaus einen C-förmigen, einen dreieckigen oder einen asymmetrischen Aufbau der Magnetfeld-erzeugenden Komponenten aufweist. Die Magnetresonanzvorrichtung 10 kann insbesondere eine dedizierte Magnetresonanzvorrichtung 10 sein, welche dazu ausgebildet ist, eine Magnetresonanzbildgebung der Kieferregion eines stehenden oder sitzenden Patienten 15 durchzuführen.

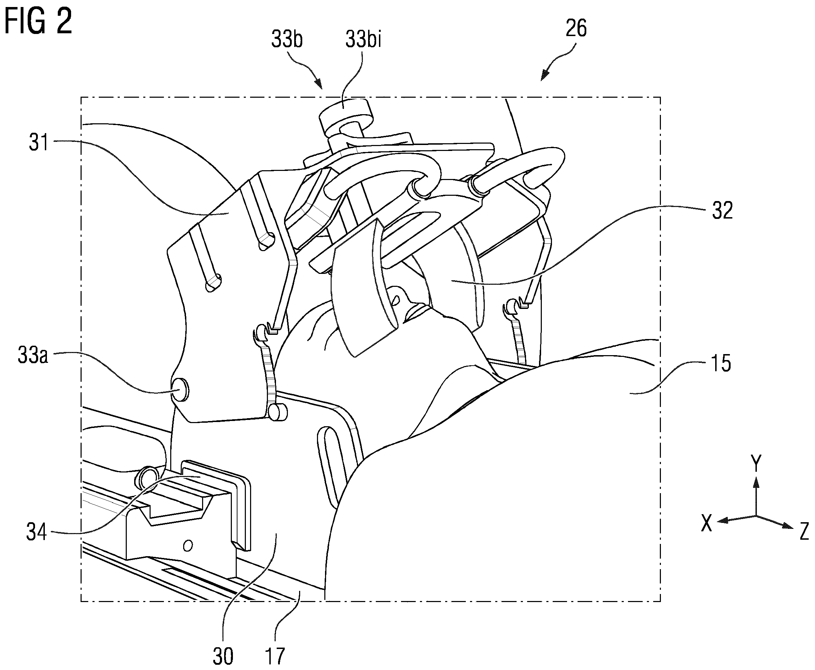

In Fig. 2 ist eine Ausführungsform der erfindungsgemäßen Lokalspule 26 dargestellt, bei welcher das Halteelement 31 gegenüber dem Basiselement 30 und/oder einer Positionierungseinheit 34 schwenkbar gelagert ist. Der Führungsmechanismus 33a weist hierfür ein Drehlager auf. Mittels des Schwenkmechanismus lässt sich das Halteelement 31 um einen Angelpunkt, welcher durch eine Position oder eine Achse des Drehlagers definiert ist, gegenüber dem Patiententisch 17 und/oder dem Patienten 15 schwenken oder verkippen.

Die Positionierungseinheit 34 ist dazu ausgebildet, die Lokalspule 26 relativ zu der Patientenlagerungsvorrichtung 16 und/oder dem Patienten 15 zu positionieren. Die Positionierungseinheit 34 kann hierfür z. B. eine Linearführung, einen Schienensystem und/oder einen vergleichbaren Führungsmechanismus aufweisen. Die Positionierungseinheit 34 ist mechanisch mit dem Basiselement 30 verbunden, welches wiederum mittels des Führungsmechanismus 33a mit dem Halteelement 31 verbunden ist.

Das Halteelement 33b weist einen Führungsmechanismus 33b auf, welcher eine relative Bewegung einer Trägerstruktur mit der Antenne 32 (im Folgenden nur Antenne 32) zu dem Halteelement 31 ermöglicht. Der Führungsmechanismus 33b ist dazu ausgebildet, die Antenne 32 entlang einer Führungsachse zu positionieren, welche bei anwendungsgemäßer Positionierung der Lokalspule 26 an der Kieferregion des Patienten 15, also beispielsweise in einem zugeklappten Zustand des Halteelements 31, im Wesentlichen parallel zu einer Schnittlinie einer Sagittalebene und einer Transversalebene des Patienten 15 ausgerichtet ist.

In einer Ausführungsform kann der Führungsmechanismus 33b Stifte oder Bolzen aufweisen, welche durch das Halteelement 31 hindurchführen und mit der Antenne 32 mechanisch verbunden sind. Der Führungsmechanismus 33b ist vorliegend dazu ausgebildet, die Antenne 32 entlang der Y-Richtung zu positionieren, wenn die Schraube 33bi gedreht wird. Die Bolzen oder Stifte des Führungsmechanismus 33b können hierbei z. B. durch Langlöcher in dem Halteelement 31 geführt sein, sodass sich die Antenne 32 auch entlang der Z-Richtung relativ zu dem Patienten 15 positionieren lässt.

Die Antenne 32 ist im gezeigten Beispiel der Fig. 2 in eine Trägerstruktur eingebettet, welche sich einer Kontur einer Oberfläche des Kopfes des Patienten 15 nachformen lässt. Vorzugsweise ist die Trägerstruktur weiterhin dazu ausgebildet, den Patienten 15 vor elektrischer Spannung und/oder einer Wärmeentwicklung durch die Antenne 32 zu schützen. Die Antenne 32 kann mehrere Antennen bzw. Signalleiter aufweisen, welche nebeneinander oder teilweise überlappend in der Trägerstruktur angeordnet sind.

In einer Ausführungsform weist das Basiselement 30 der Dentalspule 26, wie in Fig. 2 gezeigt, ein Ablageelement auf. Das Ablageelement kann beispielsweise als ein Kissen oder ein vergleichbares, elastisches Element ausgestaltet sein, welches sich einer Form einer dorsalen Seite des Kopfes des Patienten 15 anpasst. Vorzugsweise weist das Ablageelement eine zweite Antenne (nicht gezeigt) auf, welche bei anwendungsgemäßer Positionierung des Patienten 15 relativ zu dem Basiselement 30 an der dorsalen Seite des Kopfes des Patienten 15 positioniert ist. Die zweite Antenne kann elektrisch mit der Antenne 32 verbunden sein oder elektrisch von dieser getrennt sein.

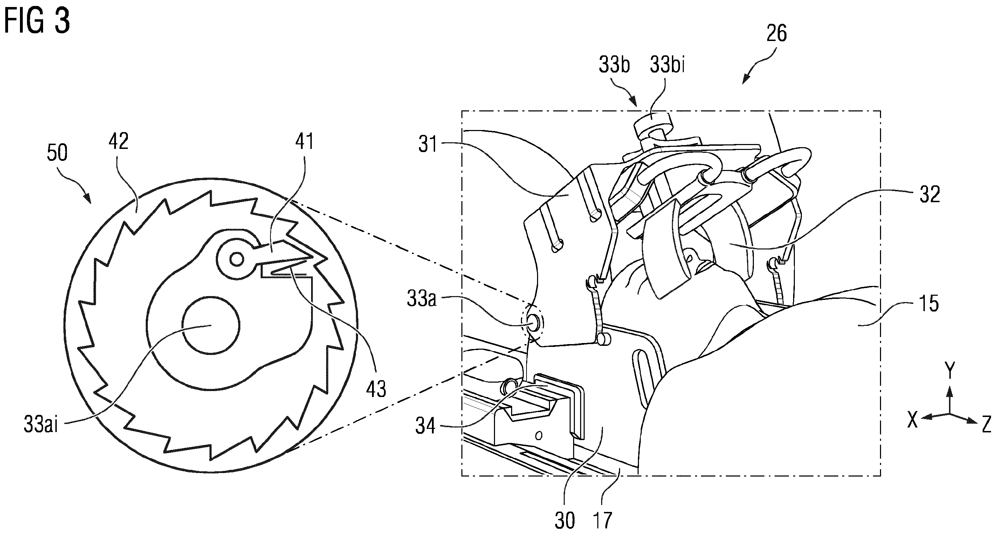

Fig. 3 zeigt eine mögliche Ausführungsform des Sicherheitsmechanismus 50, welcher vorliegend in den Führungsmechanismus 33a integriert ist. Das Drehlager weist vorliegend ein Rastelement 41 (z. B. eine erste Rastkomponente) auf, welches dazu ausgebildet ist, bei einer Schließbewegung des Halteelements 31 in das Rasterprofil 42 (z. B. eine zweite Rastkomponente) einzugreifen, um eine Schließbewegung des Haltelements 31, also eine relative Bewegung des Halteelements 31 in Richtung des Basiselements 30, zu verhindern oder zu unterbrechen. Das Rasterelement 41 kann hierfür mechanisch mit einer Welle 33ai des gezeigten Drehlagers verbunden sein. Entsprechend kann das Rasterprofil 42 derart in das Haltelement 31 integriert sein, dass es die Welle 33ai des Drehlagers entlang einer Umfangsrichtung außenumfänglich umschließt. Selbstverständlich sind verschiedene Ausführungsformen und Variationen des Sicherheitsmechanismus 50 vorstellbar, welche hier nicht im Einzelnen diskutiert werden sollen. In einem Beispiel kann das Rasterprofil 42 auch in der Welle 33ai ausgestaltet sein, während das Rastelement 41 an einem Abschnitt des Halteelements 31 und/oder des Basiselements 30 positioniert ist, welcher die Welle entlang einer Umfangsrichtung außenumfänglich umschließt.

Das Rastelement 41 ist in dem gezeigten Beispiel mittels eines Sicherungselements 43 mechanisch mit dem Führungsmechanismus 33b gekoppelt. Das Sicherungselement 43 kann z. B. eine Feder umfassen, welche durch ein Positionieren der Antenne 32 in einer vorbestimmten relativen Position zu dem Halteelement 31 gestaucht oder in eine Leerlaufposition überführt wird. Das Rastelement 41 ist wie in Fig. 3 gezeigt an dem Sicherungselement 43 gelagert und wird in der Leerlaufposition mechanisch von dem Rasterprofil 42 getrennt. Befindet sich die Antenne 32 dagegen nicht in der vorbestimmten relativen Position zu dem Haltelement 31, so verbleibt das Sicherungselement 43 wie vorliegend gezeigt in einer Haltestellung und lenkt das Rastelement 41 in Richtung des Rasterprofils 42 aus. Dadurch greift das Rastelement 41 in das Rasterprofil 42 ein und verhindert eine Schließbewegung des Halteelements 31 gegenüber dem Basiselement 30. Das Sicherungselement 43 ist vorzugsweise als eine Feder ausgestaltet, welche sich bei einer Öffnungsbewegung des Haltelements 31 gegenüber dem Basiselement 30 elastisch verformen lässt, sodass das Rastelement 41 einzelne Anschlagspunkte des Rasterprofils 42 passieren kann.

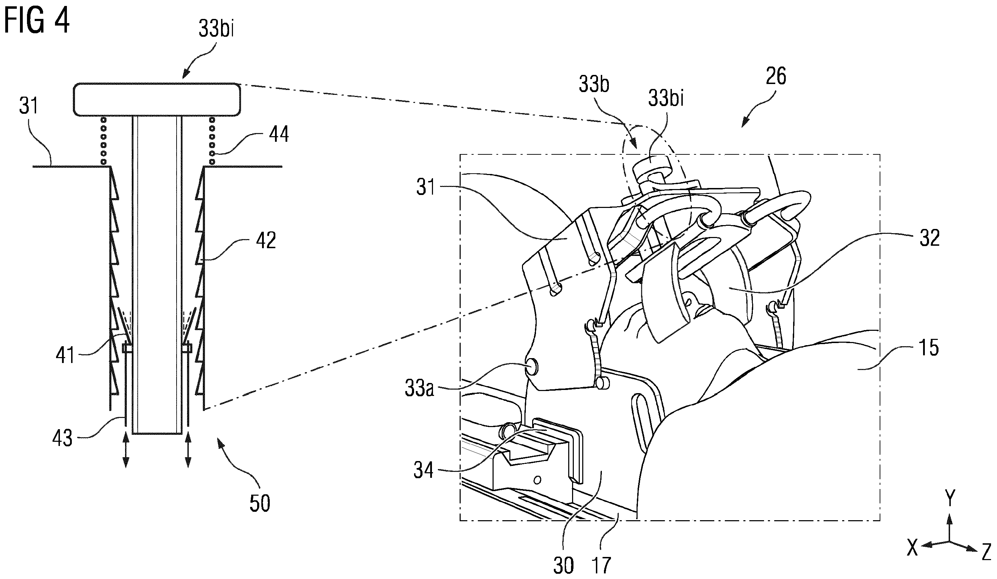

Fig. 4 zeigt eine Ausführungsform der erfindungsgemäßen Lokalspule 26, bei welcher der Führungsmechanismus 33b ein Spannelement 44 aufweist. Das Spannelement 44 ist vorliegend als eine mechanische Feder ausgeführt, welche mit einem manuellen Stellteil 33bi und einer Oberfläche des Halteelements 31 verbunden ist. Das manuelle Stellteil 33bi ist mechanisch mit der Antenne 32 verbunden und dazu ausgebildet, die Antenne 32 relativ zu dem Halteelement 31 zu bewegen. Bei einer relativen Bewegung der Antenne 32 in Richtung des Basiselements 30 mittels des manuellen Stellteils 33bi wird das Spannelement 44 gestaucht und übt eine dem Basiselement 30 entgegengerichtete Kraft auf das manuelle Stellteil 33bi mit der Antenne 32 aus.

Der Führungsmechanismus 33b umfasst vorliegend eine im Wesentlichen zylindrische Bohrung, welche auf der Innenseite ein Rasterprofil 42 (z. B. eine zweite Rastkomponente) aufweist. Der Sicherheitsmechanismus 50 weist ein Rastelement 41 (z. B. eine erste Rastkomponente) auf, welches dazu ausgebildet ist, in das Rasterprofil 42 einzugreifen und eine Bewegung des manuellen Stellteils 33bi und der Antenne 32 in einer dem Basiselement 30 abgewandten Richtung zu verhindern. Dies kann eine Positionierung der Antenne 32 in der anwendungsgemäßen Position an der Kieferregion des Patienten 15 für den Nutzer vereinfachen, da das manuelle Stellteil 33bi bereits bei dem Führen der Antenne 32 in Richtung des Patienten 15 in durch das Rasterprofil 42 vorgegebenen Schritten arretiert wird.

Das Rastelement kann wie in Fig. 3 gezeigt an einer Feder gelagert sein, welche dazu ausgebildet ist, das Rastelement 41 in Richtung des Rasterprofils 42 auszulenken. Vorliegend weist der Sicherheitsmechanismus 50 ferner ein Sicherungselement 43 auf, welches innerhalb der zylindrischen Bohrung verschiebbar gelagert ist und dazu ausgebildet ist, das Rastelement 41 mechanisch von dem Rasterprofil 42 zu trennen. Das Sicherungselement 43 kann mechanisch mit einem manuellen Schalter oder Hebel verbunden sein, welcher es dem Nutzer erlaubt, das Sicherungselement 43 manuell zu betätigen, um die Antenne 32 relativ zu dem Halteelement 31 in eine dem Basiselement 30 abgewandten Richtung zu bewegen. Vorzugsweise ist das Sicherungselement 43 jedoch mechanisch mit dem Führungsmechanismus 33a gekoppelt. Die mechanische Kopplung zwischen dem Sicherungselement 43 und dem Führungsmechanismus 33a kann derart ausgestaltet sein, dass das Sicherungselement 43 in Richtung des Rastelements 41 ausgelenkt wird und dieses mechanisch von dem Rasterprofil 42 trennt, wenn das Halteelement 31 relativ zu dem Basiselement 30 in eine der Patientenlagerungsvorrichtung abgewandte Position (also eine Öffnungsposition) bewegt wird.

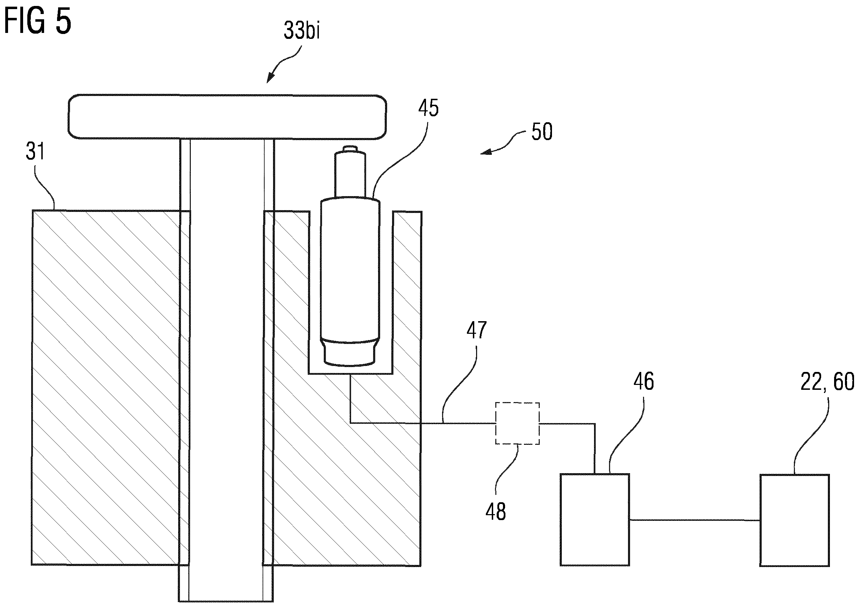

Fig. 5 zeigt eine Ausführungsform der erfindungsgemäßen Lokalspule 26, bei welcher der Sicherheitsmechanismus 50 ein als Druckfeder ausgestaltetes Stellelement 45 mit einem Antrieb 46 aufweist. Der Antrieb 46 kann, wie dargestellt, als ein pneumatischer Antrieb, aber auch als ein elektrischer oder ein hydraulischer Antrieb ausgestaltet sein. Das Stellelement 45 ist dazu ausgebildet, eine Kraft auf das manuelle Stellteil 33bi des Führungsmechanismus 33b zu übertragen. Die Kraft ist vorliegend in eine dem Basiselement 30 und/oder dem Patienten 15 entgegengesetzte Richtung ausgerichtet, sodass ein Bewegen der Antenne 32 in eine dem Basiselement 30 zugewandte Richtung mittels des Führungsmechanismus 33b erschwert oder verhindert wird. Der pneumatische Antrieb 46 weist dabei eine Signalverbindung mit einem Sensor 60 und/oder der Steuereinheit 22 der Magnetresonanzvorrichtung 10 auf. In einer Ausführungsform ist der pneumatische Antrieb 46 oder eine Steuereinheit (nicht gezeigt) des pneumatischen Antriebs 46 dazu ausgebildet, mittels der Signalverbindung ein Signal, welches eine Information über eine vorbestimmte relative Position des Halteelements 31 zu dem Basiselement 30 umfasst, zu erfassen. Die Information über die vorbestimmte relative Position des Halteelements 31 zu dem Basiselement 30 kann beispielsweise eine Information über ein Erreichen einer Endposition, eine aktuelle relative Position des Halteelements 31 zu dem Basiselement 30 und/oder eine Information über einen Ablauf einer Magnetresonanzmessung, umfassen. Der Sensor 60 kann beispielsweise als ein Abstandssensor, ein Inkrementalgeber, ein Positionsgeber, ein Kontaktgeber oder dergleichen ausgeführt sein. Vorzugsweise ist der Sensor 60 dazu ausgebildet, ein Vorliegen des Halteelements 31 in einer vorbestimmten relativen Position zu dem Basiselement 30 (und/oder einer entsprechenden Konfiguration des Führungsmechanismus 33a) zu ermitteln.

In einer weiteren Ausführungsform kann auf eine Signalverbindung mit der Steuereinheit 22 und/oder einem Sensor 60 verzichtet werden. Der Antrieb 46 weist in diesem Fall ein Arretierungselement 48 auf, welches dazu ausgebildet ist, die Fluidverbindung 47 zu dem Stellelement 45 zu unterbinden, wenn sich das Halteelement 31 in einer vorbestimmten relativen Position zu dem Basiselement 30 befindet. Das Arretierungselement 48 kann beispielsweise als ein Ventil ausgestaltet sein. Der Führungsmechanismus 33a ist

In einer alternativen Ausführungsform ist das Stellelement 45 dazu ausgebildet, die Antenne 32 in eine Ausgangsposition oder eine vorbestimmte relative Position gegenüber dem Halteelement 31 zu überführen. Hierfür kann das Stellelement 45 insbesondere auch in der zylindrischen Bohrung des Halteelements 31 integriert sein und eine Rotationsbewegung und/oder eine Translationsbewegung auf das Stellteil 33bi übertragen. Vorzugsweise ist der Antrieb 46 dabei als ein elektrischer Antrieb ausgestaltet, welcher die Antenne 32 in Abhängigkeit der Information über die vorbestimmte relative Position des Halteelements 31 zu dem Basiselement 30 positioniert. Es ist weiterhin vorstellbar, dass auf eine Implementierung eines Arretierungselements 48 verzichtet wird. Das Stellelement 45 und der Antrieb 46 können in diesem Fall dazu ausgebildet sein, die Antenne 32 automatisch in Abhängigkeit eines Signals des Sensors 60 und/oder der Steuereinheit 22 in die vorbestimmte relative Position zu dem Halteelement 31 zu überführen. Es ist weiterhin vorstellbar, dass das Stellelement 45 und der Antrieb 46 dazu ausgebildet sind, die Antenne 32 automatisch oder per Fernsteuerung durch einen Nutzer relativ zu dem Halteelement 31 zu positionieren.

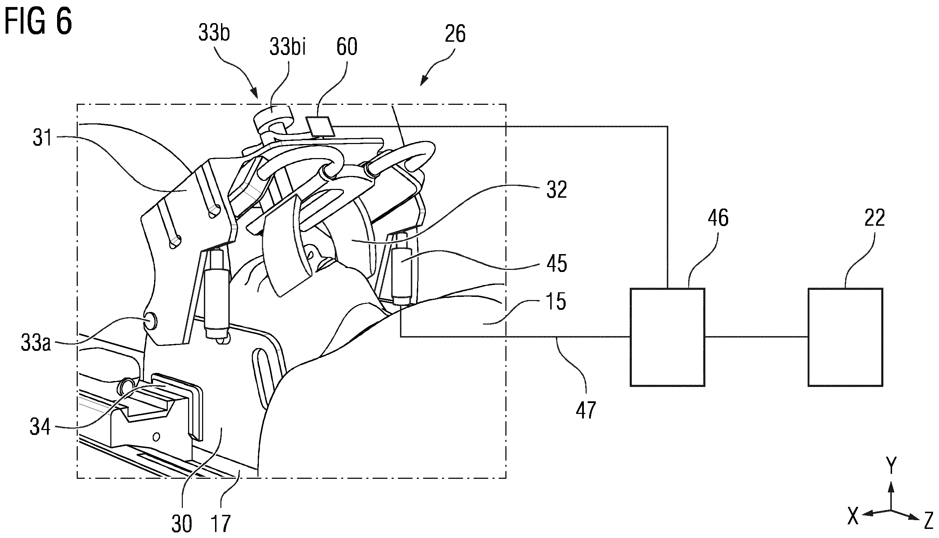

Fig. 6 zeigt eine weitere mögliche Ausführungsform der erfindungsgemäßen Lokalspule 26, bei welcher der Sicherheitsmechanismus 50 ein Stellelement 45 umfasst. Das Stellelement 45 ist vorliegend als eine Druckfeder ausgeführt, welche von einem Antrieb 46 mit einem Druck beaufschlagt wird. Der Druck ist vorzugsweise so hoch, dass ein manuelles Überführen des Halteelements 31 in die Schließposition verhindert wird.

Der Antrieb 46 weist eine Signalverbindung mit einem Sensor 60 und/oder der Steuerungseinheit 22 der Magnetresonanzvorrichtung 10 auf. Der Sensor 60 kann beispielsweise als ein Abstandssensor, ein Inkrementalgeber, ein Positionsgeber, ein Kontaktgeber oder dergleichen ausgeführt sein, welcher dazu ausgebildet ist, ein Vorliegen der Antenne 32 in einer vorbestimmten relativen Position zu dem Halteelement 31 (und/oder einer entsprechenden Konfiguration des Führungsmechanismus 33b) zu ermitteln. Bei Vorliegen einer solchen vorbestimmten Position der Antenne 32 zu dem Halteelement 31 übermittelt der Sensor 60 ein Signal an den Antrieb 46. Dieser ist entsprechend dazu ausgebildet, bei Vorliegen eines solchen Signals des Sensors 60, eine Kraftwirkung auf die Druckfeder anzupassen, sodass das Halteelement 31 manuell in die Schließposition überführt werden kann. Der Antrieb kann beispielsweise dazu ausgebildet sein, ein Ventil (vgl. Fig. 5) zu einem Bypass öffnen bzw. einen Druck in einer Leitung der Fluidverbindung 47 zu manipulieren. Es ist ebenso vorstellbar, dass der Antrieb 46 anstelle oder zusätzlich zu dem Sensor 60 eine Signalverbindung mit der Steuereinheit 22 der Magnetresonanzvorrichtung 10 aufweist. In diesem Fall kann der Antrieb 46 entsprechend einer oben beschriebenen Ausführungsform dazu ausgebildet sein, die Kraftwirkung auf die Druckfeder in Abhängigkeit eines Fortschritts der Magnetresonanzmessung anzupassen.

Selbstverständlich kann der Antrieb 46 in Fig. 6 auch als ein elektrischer Antrieb ausgestaltet sein, welcher dazu ausgebildet ist, eine relative Bewegung des Haltelements 31 zu dem Basiselement 30 in Abhängigkeit der vorbestimmten relativen Position der Antenne 32 zu dem Halteelement 31 zu begrenzen. Es ist ferner vorstellbar, dass eine Öffnungsbewegung und/oder eine Schließbewegung des Halteelements 31 mittels des Antriebs 46 automatisiert erfolgen. Beispielsweise kann ein Öffnen und/oder Schließen des Halteelements 31 gegenüber dem Basiselement 30, aber auch ein relatives Bewegen der Antenne 32 in eine Sicherheitsposition (welche eine Verletzung des Patienten ausschließt) in Abhängigkeit eines Signals des Sensors 60 und/oder der Steuereinheit 22 erfolgen.

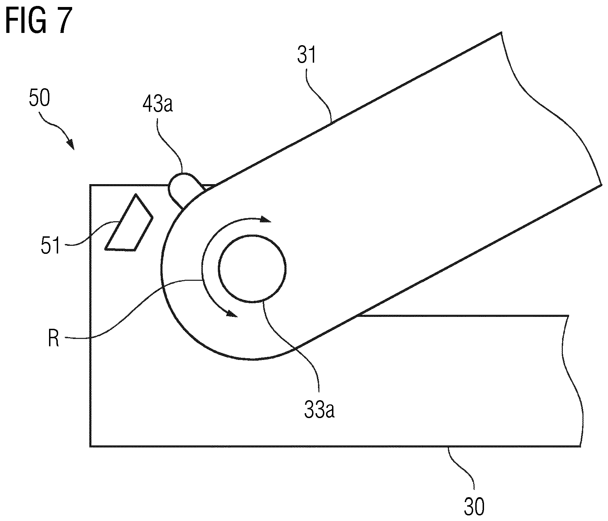

Fig. 7 zeigt eine Ausführungsform der erfindungsgemäßen Lokalspule 26 gemäß Fig. 4. Das Sicherungselement 43 weist vorliegend einen Nippel 43a auf, welcher mechanisch mit dem Sicherungselement 43 gemäß Fig. 4 gekoppelt ist. Bei einer Öffnungsbewegung des Halteelements 31 wird der Nippel 43a gegen ein Anschlagselement 51 geführt und relativ zu dem Halteelement 31 entlang der Rotationsrichtung R bewegt. Die Bewegung bzw. Verkippung des Nippels 43a bewirkt ein Auslenken des Sicherungselements 43, wodurch das Rastelement 41 von dem Rasterprofil 42 mechanisch getrennt wird und das manuelle Stellteil 33bi mittels des Spannelements 44 in die dem Basiselement 30 abgewandte Richtung bewegt wird. Selbstverständlich kann der Sicherheitsmechanismus 50 auch derart implementiert sein, dass die mechanische Trennung des Rastelements 41 von dem Rasterprofil 42 durch eine Schließbewegung des Halteelements 31 erfolgt.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen.

## Patentansprüche

1. Lokalspule (26), umfassend zumindest eine Antenne (32), ein Basiselement (30), ein Halteelement (31), einen ersten Führungsmechanismus (33a), einen zweiten Führungsmechanismus (33b) und einen Sicherheitsmechanismus (50), wobei die zumindest eine Antenne (32) dazu ausgebildet ist, hochfrequente Signale in einem Frequenz- und Leistungsbereich einer Magnetresonanzmessung zu empfangen und wobei die zumindest eine Antenne (32) mechanisch mit dem Halteelement (31) verbunden ist, wobei das Basiselement (30) dazu ausgebildet ist, das Halteelement (31) mit der zumindest einen Antenne (32) in einer anwendungsgemäßen Position an einer diagnostisch relevanten Körperregion eines Patienten (15) zu halten, wobei der erste Führungsmechanismus (33a) mechanisch mit dem Basiselement (30) und dem Halteelement (31) verbunden ist und dazu ausgebildet ist, das Halteelement (31) veränderlich gegenüber dem Basiselement (30) zu positionieren und wobei der zweite Führungsmechanismus (33b) mechanisch mit dem Halteelement (31) und der zumindest einen Antenne (32) verbunden ist und dazu ausgebildet ist, die zumindest eine Antenne (32) veränderlich gegenüber dem Halteelement (31) zu positionieren, **dadurch gekennzeichnet, dass** der Sicherheitsmechanismus (50) dazu ausgebildet ist, eine Kollision der zumindest einen Antenne (32) mit dem Patienten (15), insbesondere der diagnostisch relevanten Körperregion des Patienten (15), bei einem Überführen des Halteelements (31) von einer Öffnungsposition in eine Schließposition mittels des ersten Führungsmechanismus (33a) zu verhindern, wobei der Sicherheitsmechanismus (50) dazu ausgebildet ist, eine Bewegung des Halteelements (31) mittels des ersten Führungsmechanismus (33a) bei dem Überführen des Halteelements (31) von der Öffnungsposition in die Schließposition zu verhindern oder zu blockieren, wenn die relative Position der zumindest einen Antenne zu dem Halteelement einen vorbestimmten Grenzwert überschreitet.

2. Lokalspule (26) nach Anspruch 1, wobei der Sicherheitsmechanismus (50) eine erste Rastkomponente (41,42) aufweist, welche dazu ausgebildet ist, in eine zweite Rastkomponente (41,42) des ersten Führungsmechanismus (33a) einzugreifen und das Überführen des Halteelements (31) von der Öffnungsposition in die Schließposition zu verhindern, wobei die erste Rastkomponente (41,42) mechanisch mit einem Sicherungselement (43) verbunden ist und wobei die erste Rastkomponente (41,42) mittels des Sicherungselements (43) manuell oder automatisiert von der zweiten Rastkomponente (41,42) des ersten Führungsmechanismus (33a) trennbar ist, um das Überführen des Halteelements (31) von der Öffnungsposition in die Schließposition freizugeben.

3. Lokalspule (26) nach Anspruch 2, wobei die erste Rastkomponente (41,42) mittels des Sicherungselements (43) mechanisch mit dem zweiten Führungsmechanismus (33b) gekoppelt ist und wobei die erste Rastkomponente (41,42) mittels eines Bewegens der zumindest einen Antenne (32) in eine vorbestimmte relative Position zu dem Halteelement (31) mittels des zweiten Führungsmechanismus (33b) mechanisch von der zweiten Rastkomponente (41,42) des ersten Führungsmechanismus (33a) trennbar ist, um das Überführen des Halteelements (31) von der Öffnungsposition in die Schließposition freizugeben.

4. Lokalspule (26), umfassend zumindest eine Antenne (32), ein Basiselement (30), ein Halteelement (31), einen ersten Führungsmechanismus (33a), einen zweiten Führungsmechanismus (33b) und einen Sicherheitsmechanismus (50), wobei die zumindest eine Antenne (32) dazu ausgebildet ist, hochfrequente Signale in einem Frequenz- und Leistungsbereich einer Magnetresonanzmessung zu empfangen und wobei die zumindest eine Antenne (32) mechanisch mit dem Halteelement (31) verbunden ist, wobei das Basiselement (30) dazu ausgebildet ist, das Halteelement (31) mit der zumindest einen Antenne (32) in einer anwendungsgemäßen Position an einer diagnostisch relevanten Körperregion eines Patienten (15) zu halten, wobei der erste Führungsmechanismus (33a) mechanisch mit dem Basiselement (30) und dem Halteelement (31) verbunden ist und dazu ausgebildet ist, das Halteelement (31) veränderlich gegenüber dem Basiselement (30) zu positionieren und wobei der zweite Führungsmechanismus (33b) mechanisch mit dem Halteelement (31) und der zumindest einen Antenne (32) verbunden ist und dazu ausgebildet ist, die zumindest eine Antenne (32) veränderlich gegenüber dem Halteelement (31) zu positionieren,
**dadurch gekennzeichnet, dass** der Sicherheitsmechanismus (50) dazu ausgebildet ist, eine Kollision der zumindest einen Antenne (32) mit dem Patienten (15), insbesondere der diagnostisch relevanten Körperregion des Patienten (15), bei einem Überführen des Halteelements (31) von einer Öffnungsposition in eine Schließposition mittels des ersten Führungsmechanismus (33a) zu verhindern, wobei der Sicherheitsmechanismus (50) als eine mechanische Kopplung zwischen dem ersten Führungsmechanismus (33a) und dem zweiten Führungsmechanismus (33b) ausgestaltet ist und dazu ausgebildet ist, die zumindest eine Antenne (32) unter Ausnutzung einer Kinematik einer relativen Bewegung des Halteelements (31) zu dem Basiselement (30) in eine Sicherheitsposition zu überführen, wobei die Sicherheitsposition durch eine vorbestimmte relative Position der zumindest einen Antenne gegenüber dem Halteelement charakterisiert ist.

5. Lokalspule (26) nach Anspruch 4, wobei der zweite Führungsmechanismus (33b) ein Spannelement (44) aufweist, welches dazu ausgebildet ist, infolge einer relativen Bewegung der zumindest einen Antenne (32) zu dem Halteelement (31) in Richtung des Basiselements (30) elastisch verformt zu werden und eine der Bewegung entgegengerichtete Kraft auf die zumindest eine Antenne (32) zu übertragen, wobei der Sicherheitsmechanismus (50) eine erste Rastkomponente (41,42) aufweist, welche mechanisch mit der zumindest einen Antenne (32) verbunden ist und dazu ausgebildet ist, in eine zweite Rastkomponente (41,42) des zweiten Führungsmechanismus (33b) einzugreifen und der Kraft des Spannelements (44) entgegenzuwirken, wobei die erste Rastkomponente (41,42) mechanisch mit dem ersten Führungsmechanismus (33a) gekoppelt ist und wobei die erste Rastkomponente (41,42) mittels einer vorbestimmten relativen Bewegung des Halteelements (31) zu dem Basiselement (30) mechanisch von der zweiten Rastkomponente (41,42) des zweiten Führungsmechanismus (33b) trennbar ist, um ein Auslenken der zumindest einen Antenne (32) in eine dem Basiselement (30) abgewandte Richtung mittels des Spannelements (44) zu ermöglichen.

6. Lokalspule (26), umfassend zumindest eine Antenne (32), ein Basiselement (30), ein Halteelement (31), einen ersten Führungsmechanismus (33a), einen zweiten Führungsmechanismus (33b) und einen Sicherheitsmechanismus (50), wobei die zumindest eine Antenne (32) dazu ausgebildet ist, hochfrequente Signale in einem Frequenz- und Leistungsbereich einer Magnetresonanzmessung zu empfangen und wobei die zumindest eine Antenne (32) mechanisch mit dem Halteelement (31) verbunden ist, wobei das Basiselement (30) dazu ausgebildet ist, das Halteelement (31) mit der zumindest einen Antenne (32) in einer anwendungsgemäßen Position an einer diagnostisch relevanten Körperregion eines Patienten (15) zu halten, wobei der erste Führungsmechanismus (33a) mechanisch mit dem Basiselement (30) und dem Halteelement (31) verbunden ist und dazu ausgebildet ist, das Halteelement (31) veränderlich gegenüber dem Basiselement (30) zu positionieren und wobei der zweite Führungsmechanismus (33b) mechanisch mit dem Halteelement (31) und der zumindest einen Antenne (32) verbunden ist und dazu ausgebildet ist, die zumindest eine Antenne (32) veränderlich gegenüber dem Halteelement (31) zu positionieren,
**dadurch gekennzeichnet, dass** der Sicherheitsmechanismus (50) dazu ausgebildet ist, eine Kollision der zumindest einen Antenne (32) mit dem Patienten (15), insbesondere der diagnostisch relevanten Körperregion des Patienten (15), bei einem Überführen des Halteelements (31) von einer Öffnungsposition in eine Schließposition mittels des ersten Führungsmechanismus (33a) zu verhindern, wobei der Sicherheitsmechanismus (50) ein Stellelement (45) umfasst, welches dazu ausgebildet ist, eine Kraft auf die zumindest eine Antenne (32) zu übertragen, um die zumindest eine Antenne (32) relativ zu dem Halteelement (31) in eine dem Basiselement (30) abgewandte Richtung auszulenken, wobei die Lokalspule (26) ferner ein Arretierungselement (48) aufweist, welches mechanisch mit dem ersten Führungsmechanismus (33a) gekoppelt ist und dazu ausgebildet ist, die Übertragung der Kraft auf die zumindest eine Antenne (32) durch das Stellelement (45) zu unterbrechen, wenn sich das Basiselement (30) und das Haltelement in einer vorbestimmten relativen Position befinden.

7. Lokalspule (26), umfassend zumindest eine Antenne (32), ein Basiselement (30), ein Halteelement (31), einen ersten Führungsmechanismus (33a), einen zweiten Führungsmechanismus (33b) und einen Sicherheitsmechanismus (50), wobei die zumindest eine Antenne (32) dazu ausgebildet ist, hochfrequente Signale in einem Frequenz- und Leistungsbereich einer Magnetresonanzmessung zu empfangen und wobei die zumindest eine Antenne (32) mechanisch mit dem Halteelement (31) verbunden ist, wobei das Basiselement (30) dazu ausgebildet ist, das Halteelement (31) mit der zumindest einen Antenne (32) in einer anwendungsgemäßen Position an einer diagnostisch relevanten Körperregion eines Patienten (15) zu halten, wobei der erste Führungsmechanismus (33a) mechanisch mit dem Basiselement (30) und dem Halteelement (31) verbunden ist und dazu ausgebildet ist, das Halteelement (31) veränderlich gegenüber dem Basiselement (30) zu positionieren und wobei der zweite Führungsmechanismus (33b) mechanisch mit dem Halteelement (31) und der zumindest einen Antenne (32) verbunden ist und dazu ausgebildet ist, die zumindest eine Antenne (32) veränderlich gegenüber dem Halteelement (31) zu positionieren,
**dadurch gekennzeichnet, dass** der Sicherheitsmechanismus (50) dazu ausgebildet ist, eine Kollision der zumindest einen Antenne (32) mit dem Patienten (15), insbesondere der diagnostisch relevanten Körperregion des Patienten (15), bei einem Überführen des Halteelements (31) von einer Öffnungsposition in eine Schließposition mittels des ersten Führungsmechanismus (33a) zu verhindern, wobei der Sicherheitsmechanismus (50) ein Stellelement (45) umfasst, wobei das Stellelement (45) dazu ausgebildet ist, eine Kraft auf das Halteelement (31) zu übertragen, um eine relative Bewegung des Halteelements (31) zu dem Basiselement (30) zu begrenzen und/oder das Halteelement (31) in eine dem Basiselement (30) abgewandte Richtung auszulenken, wobei die Lokalspule (26) ferner ein Arretierungselement (48) aufweist, welches mechanisch mit dem zweiten Führungsmechanismus (33b) gekoppelt ist und dazu ausgebildet ist, die Übertragung der Kraft auf das Halteelement (31) durch das Stellelement (45) zu unterbrechen, wenn sich die zumindest eine Antenne (32) in einer vorbestimmten relativen Position zu dem Halteelement (31) befindet.

8. Lokalspule (26) nach einem der Ansprüche 6 oder 7, wobei das Stellelement (45) eine Fluidverbindung (47) mit einem pneumatischen und/oder hydraulischen Antrieb (46) aufweist und wobei das Arretierungselement (48) dazu ausgebildet ist, die Fluidverbindung (47) zwischen dem Stellelement (45) und dem pneumatischen und/oder hydraulischen Antrieb (46) zu unterbrechen.

9. Lokalspule (26), umfassend zumindest eine Antenne (32), ein Basiselement (30), ein Halteelement (31), einen ersten Führungsmechanismus (33a), einen zweiten Führungsmechanismus (33b) und einen Sicherheitsmechanismus (50), wobei die zumindest eine Antenne (32) dazu ausgebildet ist, hochfrequente Signale in einem Frequenz- und Leistungsbereich einer Magnetresonanzmessung zu empfangen und wobei die zumindest eine Antenne (32) mechanisch mit dem Halteelement (31) verbunden ist, wobei das Basiselement (30) dazu ausgebildet ist, das Halteelement (31) mit der zumindest einen Antenne (32) in einer anwendungsgemäßen Position an einer diagnostisch relevanten Körperregion eines Patienten (15) zu halten, wobei der erste Führungsmechanismus (33a) mechanisch mit dem Basiselement (30) und dem Halteelement (31) verbunden ist und dazu ausgebildet ist, das Halteelement (31) veränderlich gegenüber dem Basiselement (30) zu positionieren und wobei der zweite Führungsmechanismus (33b) mechanisch mit dem Halteelement (31) und der zumindest einen Antenne (32) verbunden ist und dazu ausgebildet ist, die zumindest eine Antenne (32) veränderlich gegenüber dem Halteelement (31) zu positionieren,
**dadurch gekennzeichnet, dass** der Sicherheitsmechanismus (50) dazu ausgebildet ist, eine Kollision der zumindest einen Antenne (32) mit dem Patienten (15), insbesondere der diagnostisch relevanten Körperregion des Patienten (15), bei einem Überführen des Halteelements (31) von einer Öffnungsposition in eine Schließposition mittels des ersten Führungsmechanismus (33a) zu verhindern, wobei der Sicherheitsmechanismus (50) ein Stellelement (45) mit einem Antrieb (46) aufweist, wobei das Stellelement (45) mechanisch mit der zumindest einen Antenne (32) oder dem Halteelement (31) verbunden ist und wobei der Antrieb (46) dazu ausgebildet ist, die zumindest eine Antenne (32) oder das Halteelement (31) in Abhängigkeit eines Steuersignals mittels des Stellelements (45) in eine dem Basiselement (30) abgewandte Richtung auszulenken.

10. Lokalspule (26) nach einem der vorhergehenden Ansprüche, wobei der erste Führungsmechanismus (33a) und/oder der zweite Führungsmechanismus (33b) als eine Linearführung, eine Teleskopführung, ein Gelenk, ein Scharnier oder ein Stecksystem ausgestaltet sind.

11. Lokalspule (26) nach Anspruch 10, wobei der erste Führungsmechanismus (33a) einen Schwenkmechanismus aufweist und wobei das Halteelement (31) mittels des Schwenkmechanismus um einen maximalen Winkel schwenkbar gegenüber dem Basiselement (30) gelagert ist, wobei die Öffnungsposition durch den maximalen Winkel zwischen dem Basiselement (30) und dem Halteelement (31) charakterisiert ist und wobei der maximale Winkel so gewählt ist, dass ein Kopf des Patienten (15) ungehindert in einer anwendungsgemäßen Relativposition zu der Lokalspule (26) positioniert werden kann.

12. Lokalspule (26) nach einem der vorhergehenden Ansprüche, ferner aufweisend eine zweite Antenne (32), wobei die zweite Antenne (32) mechanisch mit dem Basiselement (30) verbunden ist und wobei die zweite Antenne (32) bei einer anwendungsgemäßen Positionierung des Patienten (15) relativ zu dem Basiselement (30) an einer von der zumindest einen Antenne (32) abgewandten Seite des Patienten (15) positioniert ist.

13. Magnetresonanzvorrichtung (10), umfassend eine Lokalspule (26) nach einem der vorhergehenden Ansprüche, wobei die Magnetresonanzvorrichtung (10) dazu ausgebildet ist, mittels der Lokalspule (26) Magnetresonanzdaten einer diagnostisch relevanten Körperregion eines Patienten (15) zu erfassen.

## Claims

1. A local coil (26) comprising at least one antenna (32), a base element (30), a retaining element (31), a first guide mechanism (33a), a second guide mechanism (33b) and a safety mechanism (50), wherein the at least one antenna (32) is adapted to receive high-frequency signals within a frequency and power range of a magnetic resonance measurement, and wherein the at least one antenna (32) is mechanically connected to the retaining element (31), wherein the base element (30) is adapted to hold the retaining element (31) with the at least one antenna (32) in a position according to an application on a diagnostically relevant body region of a patient (15), wherein the first guide mechanism (33a) is mechanically connected to the base element (30) and the retaining element (31) and is adapted to position the retaining element (31) variably relative to the base element (30), and wherein the second guide mechanism (33b) is mechanically connected to the retaining element (31) and the at least one antenna (32) and is adapted to position the at least one antenna (32) variably relative to the retaining element (31), **characterised in that** the safety mechanism (50) is adapted to prevent a collision between the at least one antenna (32) and the patient (15), in particular the diagnostically relevant body region of the patient (15), when the retaining element (31) is moved from an open position to a closed position by means of the first guide mechanism (33a), wherein the safety mechanism (50) is adapted to prevent or block movement of the retaining element (31) by means of the first guide mechanism (33a) when the retaining element (31) is being moved from the open position to the closed position, if the relative position of the at least one antenna with respect to the retaining element exceeds a predetermined threshold value.

2. The local coil (26) according to claim 1, wherein the safety mechanism (50) comprises a first latch component (41, 42) which is adapted to engage with a second latch component (41, 42) of the first guide mechanism (33a) and to prevent the retaining element (31) from moving from the open position to the closed position, wherein the first latch component (41, 42) is mechanically connected to a securing element (43) and wherein the first latch component (41, 42) is separable manually or automatically from the second latch component (41, 42) of the first guide mechanism (33a) by means of the securing element (43), in order to allow the retaining element (31) to move from the open position to the closed position.

3. The local coil (26) according to claim 2, wherein the first latch component (41, 42) is mechanically coupled to the second guide mechanism (33b) by means of the securing element (43), and wherein the first latch component (41, 42) can be mechanically disengaged from the second latch component of the first guide mechanism (33a) by moving the at least one antenna (32) into a predetermined relative position with respect to the retaining element (31) by means of the second guide mechanism (33b), in order to allow the retaining element (31) to move from the open position to the closed position.

4. A local coil (26) comprising at least one antenna (32), a base element (30), a retaining element (31), a first guide mechanism (33a), a second guide mechanism (33b) and a safety mechanism (50), wherein the at least one antenna (32) is adapted to receive high-frequency signals within a frequency and power range of a magnetic resonance measurement, and wherein the at least one antenna (32) is mechanically connected to the retaining element (31), wherein the base element (30) is adapted to hold the retaining element (31) with the at least one antenna (32) in a position according to an application on a diagnostically relevant body region of a patient (15), wherein the first guide mechanism (33a) is mechanically connected to the base element (30) and the retaining element (31) and is adapted to position the retaining element (31) variably relative to the base element (30), and wherein the second guide mechanism (33b) is mechanically connected to the retaining element (31) and the at least one antenna (32) and is adapted to position the at least one antenna (32) variably relative to the retaining element (31), **characterised in that** the safety mechanism (50) is adapted to prevent a collision between the at least one antenna (32) and the patient (15), in particular the diagnostically relevant body region of the patient (15), when the retaining element (31) is moved from an open position to a closed position by means of the first guide mechanism (33a), wherein the safety mechanism (50) is designed as a mechanical coupling between the first guide mechanism (33a) and the second guide mechanism (33b) and is adapted to move the at least one antenna (32) into a safety position by utilising kinematics of a relative movement of the retaining element (31) with respect to the base element (30), wherein the safety position is **characterised by** a predetermined relative position of the at least one antenna with respect to the retaining element.

5. The local coil (26) according to claim 4, wherein the second guide mechanism (33b) comprises a tensioning element (44) which is adapted to be elastically deformed in the direction of the base element (30) as a result of relative movement of the at least one antenna (32) with respect to the retaining element (31), and to transmit a force opposing said movement to the at least one antenna (32), wherein the safety mechanism (50) comprises a first latch component (41, 42) which is mechanically connected to the at least one antenna (32) and is adapted to engage with a second latch component (41, 42) of the second guide mechanism (33b) and to counteract the force of the tensioning element (44), wherein the first latch component (41, 42) is mechanically coupled to the first guide mechanism (33a), and wherein the first latch component (41, 42) can be mechanically disengaged from the second latch component (41, 42) of the second guide mechanism (33b) by means of a predetermined relative movement of the retaining element (31) relative to the base element (30), in order to allow the at least one antenna (32) to be deflected in a direction away from the base element (30) by means of the tensioning element (44).

6. A local coil (26) comprising at least one antenna (32), a base element (30), a retaining element (31), a first guide mechanism (33a), a second guide mechanism (33b) and a safety mechanism (50), wherein the at least one antenna (32) is adapted to receive high-frequency signals within a frequency and power range of a magnetic resonance measurement, and wherein the at least one antenna (32) is mechanically connected to the retaining element (31), wherein the base element (30) is adapted to hold the retaining element (31) with the at least one antenna (32) in a position according to an application on a diagnostically relevant body region of a patient (15), wherein the first guide mechanism (33a) is mechanically connected to the base element (30) and the retaining element (31) and is adapted to position the retaining element (31) variably relative to the base element (30), and wherein the second guide mechanism (33b) is mechanically connected to the retaining element (31) and the at least one antenna (32) and is adapted to position the at least one antenna (32) variably relative to the retaining element (31), **characterised in that** the safety mechanism (50) is adapted to prevent a collision between the at least one antenna (32) and the patient (15), in particular the diagnostically relevant body region of the patient (15), when the retaining element (31) is moved from an open position to a closed position by means of the first guide mechanism (33a), wherein the safety mechanism (50) comprises an actuating element (45) which is adapted to transmit a force to the at least one antenna (32) in order to deflect the at least one antenna (32) relative to the retaining element (31) in a direction away from the base element (30), wherein the local coil (26) further comprises a locking element (48) which is mechanically coupled to the first guide mechanism (33a) and is configured to interrupt the transmission of the force to the at least one antenna (32) by the actuating element (45) when the base element (30) and the retaining element are in a predetermined relative position.

7. A local coil (26) comprising at least one antenna (32), a base element (30), a retaining element (31), a first guide mechanism (33a), a second guide mechanism (33b) and a safety mechanism (50), wherein the at least one antenna (32) is adapted to receive high-frequency signals within a frequency and power range of a magnetic resonance measurement, and wherein the at least one antenna (32) is mechanically connected to the retaining element (31), wherein the base element (30) is adapted to hold the retaining element (31) with the at least one antenna (32) in a position according to an application at a diagnostically relevant body region of a patient (15), wherein the first guide mechanism (33a) is mechanically connected to the base element (30) and the retaining element (31) and is adapted to position the retaining element (31) variably relative to the base element (30), and wherein the second guide mechanism (33b) is mechanically connected to the retaining element (31) and the at least one antenna (32) and is adapted to position the at least one antenna (32) variably relative to the retaining element (31), **characterised in that** the safety mechanism (50) is adapted to prevent a collision between the at least one antenna (32) and the patient (15), in particular the diagnostically relevant body region of the patient (15), when the retaining element (31) is moved from an open position to a closed position by means of the first guide mechanism (33a), wherein the safety mechanism (50) comprises an actuating element (45), wherein the actuating element (45) is adapted to transmit a force to the retaining element (31) in order to limit relative movement of the retaining element (31) relative to the base element (30) and/or to deflect the retaining element (31) in a direction away from the base e (30), wherein the local coil (26) further comprises a locking element (48) which is mechanically coupled to the second guide mechanism (33b) and is configured to interrupt the transmission of force to the retaining element (31) by the actuating element (45) when the at least one antenna (32) is in a predetermined relative position with respect to the retaining element (31).

8. The local coil (26) according to one of claims 6 or 7, wherein the actuating element (45) has a fluid connection (47) to a pneumatic and/or hydraulic drive (46) and wherein the locking element (48) is adapted to interrupt the fluid connection (47) between the actuating element (45) and the pneumatic and/or hydraulic drive (46).

9. A local coil (26) comprising at least one antenna (32), a base element (30), a retaining element (31), a first guide mechanism (33a), a second guide mechanism (33b) and a safety mechanism (50), wherein the at least one antenna (32) is adapted to receive high-frequency signals within a frequency and power range of a magnetic resonance measurement, and wherein the at least one antenna (32) is mechanically connected to the retaining element (31), wherein the base element (30) is adapted to hold the retaining element (31) with the at least one antenna (32) in a position according to an application at a diagnostically relevant body region of a patient (15), wherein the first guide mechanism (33a) is mechanically connected to the base element (30) and the retaining element (31) and is adapted to position the retaining element (31) variably relative to the base element (30), and wherein the second guide mechanism (33b) is mechanically connected to the retaining element (31) and the at least one antenna (32) and is adapted to position the at least one antenna (32) variably relative to the retaining element (31), **characterised in that** the safety mechanism (50) is adapted to prevent a collision between the at least one antenna (32) and the patient (15), in particular the diagnostically relevant body region of the patient (15), when the retaining element (31) is moved from an open position to a closed position by means of the first guide mechanism (33a), wherein the safety mechanism (50) comprises an actuating element (45) with a drive (46), wherein the actuating element (45) is mechanically connected to the at least one antenna (32) or the retaining element (31), and wherein the drive (46) is designed to deflect the at least one antenna (32) or the retaining element (31), in response to a control signal, by means of the actuating element (45) in a direction away from the base element (30).

10. The local coil (26) according to one of the preceding claims, wherein the first guide mechanism (33a) and/or the second guide mechanism (33b) are configured as a linear guide, a telescopic guide, a joint, a hinge or a plug-in system.

11. The local coil (26) according to claim 10, wherein the first guide mechanism (33a) comprises a pivoting mechanism, and wherein the retaining element (31) is mounted such that it can pivot relative to the base element (30) through a maximum angle by means of the pivoting mechanism, the open position being **characterised by** the maximum angle between the base element (30) and the retaining element (31), and wherein the maximum angle is selected such that a head of the patient (15) can be positioned unhindered in a relative position to the local coil (26) according to an application.

12. The local coil (26) according to one of the preceding claims, further comprising a second antenna (32), wherein the second antenna (32) is mechanically connected to the base element (30) and wherein, when the patient (15) is positioned relative to the base element (30) according to an application, the second antenna (32) is positioned on a side of the patient (15) facing away from the at least one antenna (32).

13. A magnetic resonance device (10) comprising a local coil (26) according to one of the preceding claims, wherein the magnetic resonance device (10) is adapted to acquire, by means of the local coil (26), magnetic resonance data from a diagnostically relevant body region of a patient (15).

## Revendications

1. Bobine locale (26) comprenant au moins une antenne (32), un élément de base (30), un élément de maintien (31), un premier mécanisme de guidage (33a), un deuxième mécanisme de guidage (33b) et un mécanisme de sécurité (50), ladite au moins une antenne (32) étant réalisée pour recevoir des signaux à haute fréquence dans une plage de fréquences et de puissances d'une mesure par résonance magnétique, et ladite au moins une antenne (32) étant reliée mécaniquement à l'élément de maintien (31), l'élément de base (30) étant réalisé pour maintenir l'élément de maintien (31) avec ladite au moins une antenne (32) dans une position adaptée à l'application au niveau d'une région corporelle d'un patient (15) pertinente en termes de diagnostic, le premier mécanisme de guidage (33a) étant relié mécaniquement à l'élément de base (30) et à l'élément de maintien (31) et étant réalisé pour positionner l'élément de maintien (31) de manière variable par rapport à l'élément de base (30), et le deuxième mécanisme de guidage (33b) étant relié mécaniquement à l'élément de maintien (31) et à ladite au moins une antenne (32) et étant réalisé pour positionner ladite au moins une antenne (32) de manière variable par rapport à l'élément de maintien (31),
**caractérisée en ce que** le mécanisme de sécurité (50) est réalisé pour empêcher une collision de ladite au moins une antenne (32) avec le patient (15), en particulier avec la région corporelle du patient (15) pertinente en termes de diagnostic, lors d'un passage de l'élément de maintien (31) depui une position d'ouverture jusque dans une position de fermeture au moyen du premier mécanisme de guidage (33a), le mécanisme de sécurité (50) étant réalisé pour empêcher ou bloquer un déplacement de l'élément de maintien (31) au moyen du premier mécanisme de guidage (33a) lors du passage de l'élément de maintien (31) depuis la position d'ouverture jusque dans la position de fermeture, quand la position relative de ladite au moins une antenne par rapport à l'élément de maintien dépasse une valeur limite prédéterminée.

2. Bobine locale (26) selon la revendication 1, le mécanisme de sécurité (50) présentant un premier composant d'encliquetage (41, 42) qui est réalisé pour s'engager dans un deuxième composant d'encliquetage (41, 42) du premier mécanisme de guidage (33a) et empêcher le passage de l'élément de maintien (31) depuis la position d'ouverture jusque dans la position de fermeture, le premier composant d'encliquetage (41, 42) étant relié mécaniquement à un élément de sécurité (43) et le premier composant d'encliquetage (41, 42) pouvant être séparé manuellement ou automatiquement du deuxième composant d'encliquetage (41, 42) du premier mécanisme de guidage (33a) au moyen de l'élément de sécurité (43) afin de libérer le passage de l'élément de maintien (31) depuis la position d'ouverture jusque dans la position de fermeture.

3. Bobine locale (26) selon la revendication 2, le premier composant d'encliquetage (41, 42) étant couplé mécaniquement au deuxième mécanisme de guidage (33b) au moyen de l'élément de verrouillage (43), et le premier composant d'encliquetage (41, 42) pouvant être séparé mécaniquement du deuxième composant d'encliquetage (41, 42) du premier mécanisme de guidage (33a) au moyen d'un déplacement de ladite au moins une antenne (32) dans une position relative prédéterminée par rapport à l'élément de maintien (31) au moyen du deuxième mécanisme de guidage (33b) afin de libérer le passage de l'élément de maintien (31) depuis la position d'ouverture jusque dans la position de fermeture.

4. Bobine locale (26) comprenant au moins une antenne (32), un élément de base (30), un élément de maintien (31), un premier mécanisme de guidage (33a), un deuxième mécanisme de guidage (33b) et un mécanisme de sécurité (50), ladite au moins une antenne (32) étant réalisée pour recevoir des signaux à haute fréquence dans une plage de fréquences et de puissances d'une mesure par résonance magnétique, et ladite au moins une antenne (32) étant reliée mécaniquement à l'élément de maintien (31), l'élément de base (30) étant réalisé pour maintenir l'élément de maintien (31) avec ladite au moins une antenne (32) dans une position adaptée à l'application sur une région corporelle d'un patient (15) pertinente en termes de diagnostic, le premier mécanisme de guidage (33a) étant relié mécaniquement à l'élément de base (30) et à l'élément de maintien (31) et étant réalisé pour positionner l'élément de maintien (31) de manière variable par rapport à l'élément de base (30), et le deuxième mécanisme de guidage (33b) étant relié mécaniquement à l'élément de maintien (31) et à ladite au moins une antenne (32) et étant réalisé pour positionner de manière variable ladite au moins une antenne (32) par rapport à l'élément de maintien (31),
**caractérisée en ce que** le mécanisme de sécurité (50) est réalisé pour empêcher une collision de ladite au moins une antenne (32) avec le patient (15), en particulier avec la région corporelle du patient (15) pertinente en termes de diagnostic, lors d'un passage de l'élément de maintien (31) depuis une position d'ouverture jusque dans une position de fermeture au moyen du premier mécanisme de guidage (33a), le mécanisme de sécurité (50) étant réalisé comme un couplage mécanique entre le premier mécanisme de guidage (33a) et le deuxième mécanisme de guidage (33b) et étant réalisé pour amener ladite au moins une antenne (32) dans une position de sécurité en exploitant la cinématique d'un déplacement relatif de l'élément de maintien (31) par rapport à l'élément de base (30), la position de sécurité étant **caractérisée par** une position relative prédéterminée de ladite au moins une antenne par rapport à l'élément de maintien.

5. Bobine locale (26) selon la revendication 4, le deuxième mécanisme de guidage (33b) présentant un élément de serrage (44) qui est réalisé pour être déformé élastiquement en direction de l'élément de base (30) à la suite d'un déplacement relatif de ladite au moins une antenne (32) par rapport à l'élément de maintien (31) et transmettre à ladite au moins une antenne (32) une force à l'encontre du déplacement, le mécanisme de sécurité (50) présentant un premier composant d'encliquetage (41, 42) qui est relié mécaniquement à ladite au moins une antenne (32) et qui est réalisé pour s'engager dans un deuxième composant d'encliquetage (41, 42) du deuxième mécanisme de guidage (33b) et contrecarrer la force de l'élément de serrage (44), le premier composant d'encliquetage (41, 42) étant couplé mécaniquement au premier mécanisme de guidage (33a) et le premier composant d'encliquetage (41, 42) pouvant être séparé mécaniquement du deuxième composant d'encliquetage (41, 42) du deuxième mécanisme de guidage (33b) au moyen d'un déplacement relatif prédéterminé de l'élément de maintien (31) par rapport à l'élément de base (30) afin de permettre une déviation de ladite au moins une antenne (32) dans une direction détournée l'élément de base (30) au moyen de l'élément de serrage (44).

6. Bobine locale (26) comprenant au moins une antenne (32), un élément de base (30), un élément de maintien (31), un premier mécanisme de guidage (33a), un deuxième mécanisme de guidage (33b) et un mécanisme de sécurité (50), ladite au moins une antenne (32) étant réalisée pour recevoir des signaux à haute fréquence dans une plage de fréquences et de puissances d'une mesure par résonance magnétique, et ladite au moins une antenne (32) étant reliée mécaniquement à l'élément de maintien (31), l'élément de base (30) étant réalisé pour maintenir l'élément de maintien (31) avec ladite au moins une antenne (32) dans une position adaptée à l'application sur une région corporelle d'un patient (15) pertinente en termes de diagnostic, le premier mécanisme de guidage (33a) étant relié mécaniquement à l'élément de base (30) et à l'élément de maintien (31) et étant réalisé pour positionner l'élément de maintien (31) de manière variable par rapport à l'élément de base (30), et le deuxième mécanisme de guidage (33b) étant relié mécaniquement à l'élément de maintien (31) et à ladite au moins une antenne (32) et étant réalisé pour positionner ladite au moins une antenne (32) de manière variable par rapport à l'élément de maintien (31),
**caractérisée en ce que** le mécanisme de sécurité (50) est réalisé pour empêcher une collision de ladite au moins une antenne (32) avec le patient (15), en particulier avec la région corporelle du patient (15) pertinente en termes de diagnostic, lors d'un passage de l'élément de maintien (31) depuis une position d'ouverture jusque dans une position de fermeture au moyen du premier mécanisme de guidage (33a), le mécanisme de sécurité (50) comprenant un élément de réglage (45) qui est réalisé pour transmettre une force à ladite au moins une antenne (32) afin de dévier ladite au moins une antenne (32) par rapport à l'élément de maintien (31) dans une direction détournée de l'élément de base (30), la bobine locale (26) présentant en outre un élément de blocage (48) qui est couplé mécaniquement au premier mécanisme de guidage (33a) et qui est réalisé pour interrompre la transmission de la force à ladite au moins une antenne (32) par l'élément de réglage (45) quand l'élément de base (30) et l'élément de maintien se trouvent dans une position relative prédéterminée.

7. Bobine locale (26) comprenant au moins une antenne (32), un élément de base (30), un élément de maintien (31), un premier mécanisme de guidage (33a), un deuxième mécanisme de guidage (33b) et un mécanisme de sécurité (50), ladite au moins une antenne (32) étant réalisée pour recevoir des signaux à haute fréquence dans une plage de fréquences et de puissances d'une mesure par résonance magnétique, et ladite au moins une antenne (32) étant reliée mécaniquement à l'élément de maintien (31), l'élément de base (30) étant réalisé pour maintenir l'élément de maintien (31) avec ladite au moins une antenne (32) dans une position adaptée à l'application au niveau d'une région corporelle d'un patient (15) pertinente en termes de diagnostic, le premier mécanisme de guidage (33a) étant relié mécaniquement à l'élément de base (30) et à l'élément de maintien (31) et étant réalisé pour positionner l'élément de maintien (31) de manière variable par rapport à l'élément de base (30), et le deuxième mécanisme de guidage (33b) étant relié mécaniquement à l'élément de maintien (31) et à ladite au moins une antenne (32) et étant réalisé pour positionner ladite au moins une antenne (32) de manière variable par rapport à l'élément de maintien (31),
**caractérisée en ce que** le mécanisme de sécurité (50) est réalisé pour empêcher une collision de ladite au moins une antenne (32) avec le patient (15), en particulier avec la région corporelle du patient (15) pertinente en termes de diagnostic, lors d'un passage de l'élément de maintien (31) depuis une position d'ouverture jusque dans une position de fermeture au moyen du premier mécanisme de guidage (33a), le mécanisme de sécurité (50) comprenant un élément de réglage (45), l'élément de réglage (45) étant réalisé pour transmettre une force à l'élément de maintien (31) afin de limiter un déplacement relatif de l'élément de maintien (31) par rapport à l'élément de base (30) et/ou de dévier l'élément de maintien (31) dans une direction détournée de l'élément de base (30), la bobine locale (26) présentant en outre un élément de blocage (48) qui est couplé mécaniquement au deuxième mécanisme de guidage (33b) et est réalisé pour interrompre la transmission de la force à l'élément de maintien (31) par l'élément de réglage (45) quand ladite au moins une antenne (32) se trouve dans une position relative prédéterminée par rapport à l'élément de maintien (31).

8. Bobine locale (26) selon l'une des revendications 6 ou 7, l'élément de réglage (45) présentant une liaison fluidique (47) avec un entraînement pneumatique et/ou hydraulique (46) et l'élément de blocage (48) étant réalisé pour interrompre la liaison fluidique (47) entre l'élément de réglage (45) et l'entraînement pneumatique et/ou hydraulique (46).

9. Bobine locale (26) comprenant au moins une antenne (32), un élément de base (30), un élément de maintien (31), un premier mécanisme de guidage (33a), un deuxième mécanisme de guidage (33b) et un mécanisme de sécurité (50), ladite au moins une antenne (32) étant réalisée pour recevoir des signaux à haute fréquence dans une plage de fréquences et de puissances d'une mesure par résonance magnétique, et ladite au moins une antenne (32) étant reliée mécaniquement à l'élément de maintien (31), l'élément de base (30) étant réalisé pour relier l'élément de maintien (31) à ladite au moins une antenne (32) dans une position adaptée à l'application sur une région corporelle d'un patient (15) pertinente en termes de diagnostic, le premier mécanisme de guidage (33a) étant relié mécaniquement à l'élément de base (30) et à l'élément de maintien (31) et étant réalisé pour positionner l'élément de maintien (31) de manière variable par rapport à l'élément de base (30), et le deuxième mécanisme de guidage (33b) étant relié mécaniquement à l'élément de maintien (31) et à ladite au moins une antenne (32) et étant réalisé pour positionner de manière variable ladite au moins une antenne (32) par rapport à l'élément de maintien (31), **caractérisée en ce que** le mécanisme de sécurité (50) est réalisé pour empêcher une collision de ladite au moins une antenne (32) avec le patient (15), en particulier avec la région corporelle du patient (15) pertinente en termes de diagnostic, lors d'un passage de l'élément de maintien (31) depuis une position d'ouverture jusque dans une position de fermeture au moyen du premier mécanisme de guidage (33a), le mécanisme de sécurité (50) présentant un élément de réglage (45) avec un entraînement (46), l'élément de réglage (45) étant relié mécaniquement à ladite au moins une antenne (32) ou à l'élément de maintien (31), et l'entraînement (46) étant réalisé pour dévier ladite au moins une antenne (32) ou ledit élément de maintien (31), en fonction d'un signal de commande au moyen de l'élément de réglage (45), dans une direction détournée de l'élément de base (30).

10. Bobine locale (26) selon l'une des revendications précédentes, le premier mécanisme de guidage (33a) et/ou le deuxième mécanisme de guidage (33b) étant configurés sous la forme d'un guidage linéaire, d'un guidage télescopique, d'une articulation, d'une charnière ou d'un système d'emboîtement.

11. Bobine locale (26) selon la revendication 10, le premier mécanisme de guidage (33a) présentant un mécanisme de pivotement et l'élément de maintien (31) étant monté de manière à pouvoir pivoter d'un angle maximum par rapport à l'élément de base (30) au moyen du mécanisme de pivotement, la position d'ouverture étant **caractérisée par** l'angle maximum entre l'élément de base (30) et l'élément de maintien (31), et l'angle maximum étant choisi de telle sorte que la tête du patient (15) peut être positionnée sans entrave dans une position relative adaptée à l'application par rapport à la bobine locale (26).

12. Bobine locale (26) selon l'une des revendications précédentes, comprenant en outre une deuxième antenne (32), la deuxième antenne (32) étant reliée mécaniquement à l'élément de base (30) et la deuxième antenne (32), lors d'un positionnement, adapté à l'application, du patient (15) par rapport à l'élément de base (30), étant positionnée sur un côté du patient (15) détourné de ladite au moins une antenne (32).

13. Dispositif à résonance magnétique (10) comprenant une bobine locale (26) selon l'une des revendications précédentes, le dispositif à résonance magnétique (10) étant réalisé pour acquérir, au moyen de la bobine locale (26), des données de résonance magnétique d'une région corporelle d'un patient (15) pertinente en termes de diagnostic.
